# EUROPEAN PATENT APPLICATION

(11) **EP 4 704 029 A1**
(43) Date of publication of application: **04.03.2026**
(21) Application number: 25197922.5
(22) Date of filing: 25.08.2025
(51) Int. Cl.: G06T 7/00, A61B 6/03, A61B 6/46, A61B 6/00

(54) **APPARATUS, METHOD AND PROGRAM TO FACILITATE BIOLOGICAL FEATURE RECOGNITION IN DENTITION CONSTITUENT TISSUE**

(30) Priority: 26.08.2024 JP 2024144057
(71) Applicant: J. MORITA MANUFACTURING CORPORATION, Fushimi-ku, Kyoto-shi, Kyoto 6128533 (JP)
(72) Inventor: NISHIMURA, Yu, Kyoto, 612-8533 (JP)
(74) Representative: Müller Hoffmann & Partner

(57) **Abstract**

An image processing apparatus displays, on a display 22, a biological feature region arrangement developed image Eq in which at least one of a plurality of annotation images Q respectively indicating a plurality of biological feature regions is superimposed on a dentition developed image Ep such that the annotation images are located at corresponding positions of the plurality of biological feature region corresponding positions. Detail observation images at the plurality of three-dimensional positions are respectively generated on the basis of the three-dimensional image data, a focus order of the plurality of biological feature regions is determined according to a predetermined rule, and when the biological feature region arrangement developed image is displayed, it is indicated that the biological feature regions are subjected to focusing in the focus order by a change in the annotation images and the detail observation images corresponding to focusing of the biological feature regions are displayed on the display.

## Description

### BACKGROUND

### Technical Field

The present disclosure relates to a technology for making it easy to recognize a biological feature region.

### Description of the Background Art

Japanese Translation of PCT International Application Publication No. 2021-528751 discloses a technology of displaying a detected region of interest on a computed tomography (CT) scan image.

Japanese Patent Application Laid-Open No. 2008-229322 discloses a technology of using X-ray projection data of a maxillofacial region obtained by X-ray CT imaging to obtain a panoramic tomographic image of a dentition.

Japanese Patent Application Laid-Open No. 2021-174394 discloses a technology of performing lesion detection by inputting a CT image to a learned model.

As in Japanese Translation of PCT International Application Publication No. 2021-528751, according to the technology of displaying the detected region of interest on the CT scan image, it may take time and effort to search for the CT scan image displaying the region of interest. In addition, in a case where a plurality of regions of interest are present, it is also required to make it easy to recognize the plurality of regions of interest without omission.

### SUMMARY

Therefore, an object of the present disclosure is to make it easy to recognize a plurality of biological feature regions without omission.

An image processing apparatus is an image processing apparatus that processes image data obtained by computed tomography (CT) imaging of dentition constituent tissue to generate an image for diagnosis, the image processing apparatus including: a storage that stores the image data; a processor; and a display that displays an observation image on the basis of output of the processor, wherein the processor generates three-dimensional image data of the dentition constituent tissue on the basis of the image data, detects a plurality of biological feature regions in the dentition constituent tissue on the basis of the image data, identifies a plurality of three-dimensional positions at which the plurality of biological feature regions are respectively present in a coordinate system of the three-dimensional image data, generates a dentition developed image in which the dentition constituent tissue is developed on the basis of the three-dimensional image data, identifies a plurality of biological feature region corresponding positions respectively corresponding to the plurality of three-dimensional positions in the dentition developed image, displays, on the display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions, generates detail observation images at the plurality of three-dimensional positions, respectively, on the basis of the three-dimensional image data, determines a focus order of the plurality of biological feature regions according to a predetermined rule, and when displaying the biological feature region arrangement developed image, indicates that the biological feature regions are subjected to focusing in the focus order by a change in the annotation images and displays, on the display, the detail observation images corresponding to focusing of the biological feature regions.

An image processing method is an image processing method of processing image data obtained by computed tomography (CT) imaging of dentition constituent tissue to generate an image for diagnosis, the method including: generating three-dimensional image data of the dentition constituent tissue on the basis of the image data; detecting a plurality of biological feature regions in the dentition constituent tissue on the basis of the image data; identifying a plurality of three-dimensional positions at which the plurality of biological feature regions are respectively present in a coordinate system of the three-dimensional image data; generating a dentition developed image in which the dentition constituent tissue is developed on the basis of the three-dimensional image data; identifying a plurality of biological feature region corresponding positions respectively corresponding to the plurality of three-dimensional positions in the dentition developed image; displaying, on a display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions; generating detail observation images at the plurality of three-dimensional positions, respectively, on the basis of the three-dimensional image data; determining a focus order of the plurality of biological feature regions according to a predetermined rule; and when displaying the biological feature region arrangement developed image, indicating that the biological feature regions are subjected to focusing in the focus order by a change in the annotation images and displaying, on the display, the detail observation images corresponding to focusing of the biological feature regions.

A program is a program for processing image data obtained by computed tomography (CT) imaging of dentition constituent tissue to generate an image for diagnosis, the program being configured to cause a computer to execute processing of: generating three-dimensional image data of the dentition constituent tissue on the basis of the image data; detecting a plurality of biological feature regions in the dentition constituent tissue on the basis of the image data; identifying a plurality of three-dimensional positions at which the plurality of biological feature regions are respectively present in a coordinate system of the three-dimensional image data; generating a dentition developed image in which the dentition constituent tissue is developed on the basis of the three-dimensional image data; identifying a plurality of biological feature region corresponding positions respectively corresponding to the plurality of three-dimensional positions in the dentition developed image; displaying, on the display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions; generating detail observation images at the plurality of three-dimensional positions, respectively, on the basis of the three-dimensional image data; determining a focus order of the plurality of biological feature regions according to a predetermined rule; and when displaying the biological feature region arrangement developed image, indicating that the biological feature regions are subjected to focusing in the focus order by a change in the annotation images and displaying, on the display, the detail observation images corresponding to focusing of the biological feature regions.

According to the present disclosure, it can be made easy to recognize a plurality of biological feature regions without omission.

These and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a schematic diagram illustrating an image processing apparatus and a CT imaging apparatus according to a preferred embodiment;
FIG. 2 is a block diagram illustrating electrical configurations of the image processing apparatus and the CT imaging apparatus;
FIG. 3 is a functional block diagram of an arithmetic circuit;
FIG. 4 is a flowchart illustrating an example of processing in the image processing apparatus;
FIG. 5 is an explanatory diagram illustrating a field of view (FOV) region;
FIG. 6 is an explanatory diagram illustrating an example of a region in which dentition constituent tissue spreads;
FIG. 7 is an explanatory diagram illustrating positional relationships between the dentition constituent tissue and lesion regions in the FOV region;
FIG. 8 is an explanatory diagram illustrating examples of coordinates of the lesion regions in the dentition constituent tissue;
FIG. 9 is an explanatory diagram illustrating an example of processing of developing the dentition constituent tissue in a dentition developed image;
FIG. 10 is a diagram illustrating a biological feature region arrangement developed image;
FIG. 11 is a flowchart illustrating a specific example of processing of step S7 in FIG. 4;
FIG. 12 is a flowchart illustrating a specific example of observation display processing in FIG. 11;
FIG. 13 is a diagram illustrating a display example of a detail observation image and a biological feature region arrangement developed image;
FIG. 14 is a flowchart illustrating an example of processing on the detail observation image in FIG. 12;
FIG. 15 is an explanatory diagram illustrating an example of moving operation processing of the detail observation image;
FIG. 16 is an explanatory diagram illustrating an example of a change in annotation images according to a focus order;
FIG. 17 is an explanatory diagram illustrating a modification of the annotation images;
FIG. 18 is an explanatory diagram illustrating a focus order according to a first modification;
FIG. 19 is a diagram illustrating a biological feature region arrangement developed image according to a second modification;
FIG. 20 is a diagram illustrating a biological feature region arrangement developed image according to a third modification;
FIG. 21 is an explanatory diagram illustrating a state in which a head is positioned by a head holder in a fourth modification;
FIG. 22 is an explanatory diagram illustrating a positional relationship between the head holder and the dentition constituent tissue;
FIG. 23 is a schematic diagram illustrating an image processing apparatus according to a fifth modification; and
FIG. 24 is a block diagram illustrating an electrical configuration of the image processing apparatus according to the fifth modification.

### DESCRIPTION OF THE PREFERRED EMBODIMENTS

### {Preferred embodiment}

Hereinafter, an image processing apparatus, an image processing method, and a program according to a preferred embodiment will be described.

### <Configuration including image processing apparatus and CT imaging apparatus>

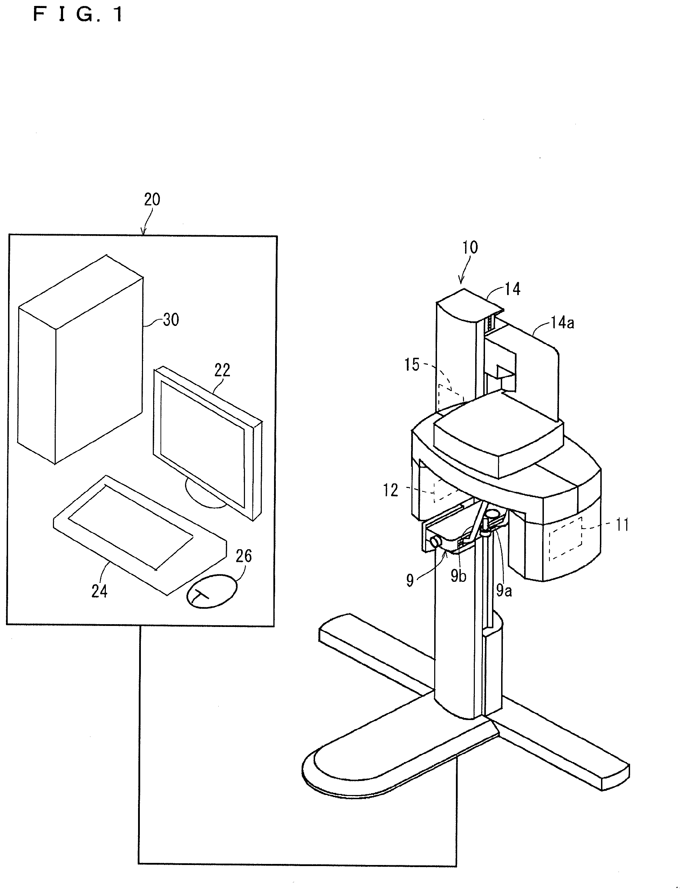

FIG. 1 is a schematic diagram illustrating an image processing apparatus 20 connected to a CT imaging apparatus 10.

The CT imaging apparatus 10 is an apparatus that obtains image data by performing computed tomography (CT) imaging of dentition constituent tissue. The obtained image data includes data on the dentition constituent tissue. The image processing apparatus 20 processes the image data to generate an image for diagnosis.

For example, the CT imaging apparatus 10 includes an X-ray generator 11, an X-ray detector 12, a turning arm 13, a support pillar 14, and an imaging processing unit 15.

In a space where the CT imaging apparatus 10 exists, three-dimensional coordinates are set for arithmetic operation. As the three-dimensional coordinates, using, for example, the orientation of a subject positioned for imaging as a reference, a Z direction along the body axis direction, an X direction orthogonal to the Z direction and along the right-left direction of the subject, and a Y direction orthogonal to the Z direction and the X direction and along the front-back direction of the subject are set. In the present preferred embodiment, since the subject is positioned at a test position in the standing position, the Z direction is a direction perpendicular to the floor surface from which the support pillar 14 extends.

The X-ray generator 11 includes an X-ray tube and is configured to be able to emit an X-ray beam toward the subject. The X-ray detector 12 includes an X-ray detection sensor. The X-ray emitted from the X-ray generator 11 passes through the subject and is detected by the X-ray detector 12.

The turning arm 13 is, for example, a member formed in a U-shape opening downward. The X-ray generator 11 and the X-ray detector 12 are supported at both ends of the turning arm 13 while facing each other. The subject may be placed between the X-ray generator 11 and the X-ray detector 12. The subject is a human body part including the dentition constituent tissue, that is, a head including a jaw.

The support pillar 14 is erected so as to extend along the gravity direction (vertical direction). A cantilever arm 14a is supported by this support pillar 14 so as to be movable up and down. The turning arm 13 is turnably supported by the cantilever arm 14a. In accordance with a height position of the head, a height position of the turning arm 13 is adjusted along the support pillar 14.

The turning arm 13 is rotated with the head of the subject positioned between both ends of the turning arm 13. As a result, the X-ray generator 11 and the X-ray detector 12 rotate around the head. Consequently, the X-ray CT imaging on the head is performed, and the image data is obtained.

For example, when the turning arm 13 turns, the X-ray imaging is performed for each minute turning angle. As a result, the X-ray projection image data (frame data) for each minute turning angle is obtained. Three-dimensional volume data of the subject is generated by being based on an X-ray projection image data group (frame data group) subjected to imaging at different turning angles. This three-dimensional volume data is three-dimensional image data indicating the distribution of the X-ray absorption rate of the subject in the three-dimensional coordinate system.

The CT imaging apparatus 10 may include a head holder 9. The head holder 9 is a part that holds a head that is an imaging target. The head holder 9 may include a chin rest 9a that supports the chin. The chin rest 9a can support the front part and the lower part of the jaw of the head. As a result, the head is positioned at a fixed position in the front-back direction and the up-down direction. The head holder 9 may include a both-sides holder 9b that positions the head from both sides. The both-sides holder 9b may be, for example, an ear rod in contact with both ears of the head. The head is positioned at a fixed position in the right-left direction by the both-sides holder 9b.

The image processing apparatus 20 processes the image data obtained by CT imaging to generate an image for diagnosis. The image data obtained by the CT imaging may be an X-ray projection image data group for each minute turning angle or may be three-dimensional volume data. In the present preferred embodiment, an example in which the image data obtained by the CT imaging is an X-ray projection data group for each minute turning angle will be described.

The image processing apparatus 20 includes a processing unit 30, a display 22, and a user interface 24, 26.

The processing unit 30 is configured by a computer, a workstation, or the like. The processing unit 30 is connected to the imaging processing unit 15 of the CT imaging apparatus 10 in a wired or wireless manner, and can transmit and receive various data to and from the imaging processing unit 15. In the present preferred embodiment, the processing unit 30 can receive image data obtained by CT imaging from the imaging processing unit 15. Some or all of the functions of the processing unit 30 may be implemented by a cloud server.

The display 22 is, for example, a liquid crystal display, an organic electroluminescence (EL) display, or the like, and is connected to the processing unit 30 in a wired or wireless manner. The display 22 can display an image for diagnosis on the basis of the output for display of the processing unit 30.

The user interface 24, 26 is an apparatus that receives instructions from the user of the image processing apparatus 20. The user interface 24 may be, for example, a switch device such as a keyboard. The user interface 26 may be, for example, a pointer device such as a mouse. When the user interface is a switch device, a user's instruction can be input by the switch device alone. When the user interface is a pointer device, a user's instruction can be input by an operation on texts or an image displayed on the display 22. The user interface may be a touch panel.

### <Electrical configurations of image processing apparatus and CT imaging apparatus>

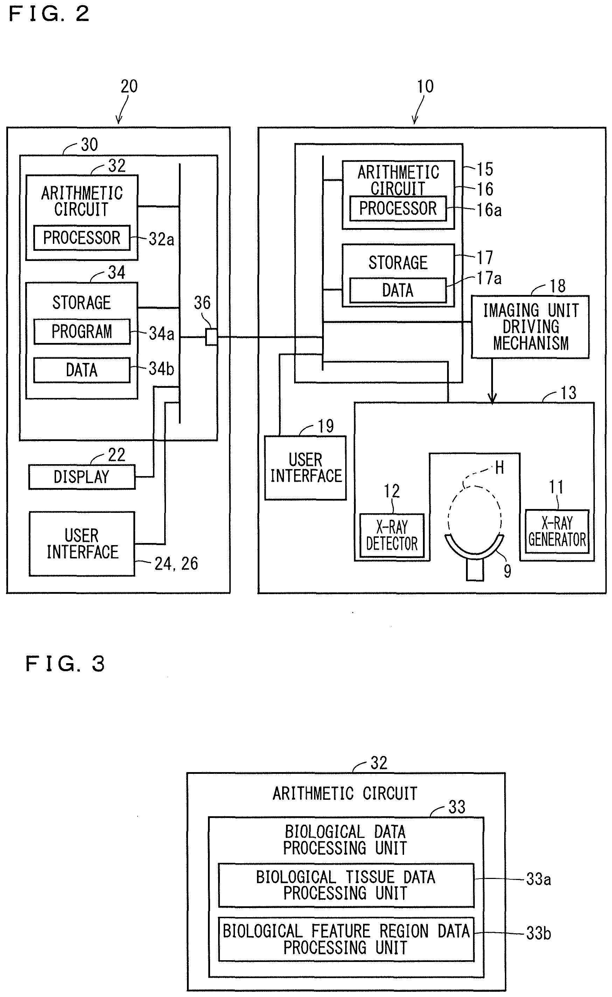

FIG. 2 is a block diagram illustrating electrical configurations of the image processing apparatus 20 and the CT imaging apparatus 10.

The CT imaging apparatus 10 includes the imaging processing unit 15, an imaging unit driving mechanism 18, and a user interface 19.

The imaging processing unit 15 is configured by a computer including an arithmetic circuit 16 and a storage 17.

The arithmetic circuit 16 includes a processor 16a. The processor 16a may be a central processing unit (CPU). The processor 16a may include a graphics processing unit (GPU).

The storage 17 is configured by a non-volatile storage such as a flash memory or a hard disk device. The storage 17 may be a storage circuit. The storage 17 stores a program. In the program, a procedure for the CT imaging apparatus 10 to perform the CT imaging is described.

The imaging unit driving mechanism 18 is connected to the imaging processing unit 15. The imaging unit driving mechanism 18 includes a turning driving mechanism for turning the turning arm 13. The turning driving mechanism includes an actuator and a transmission mechanism. The actuator is an electric motor or the like that generates a turning driving force. The transmission mechanism is a gear, a pulley, or the like that transmits rotational driving force of the actuator. The rotational driving force of the actuator is transmitted to the turning arm 13 via the transmission mechanism, and the turning arm 13 turns at a timing and a turning speed according to a command from the imaging processing unit 15.

The user interface 19 is an interface for giving an instruction to the CT imaging apparatus 10, and is a switch device, a pointer device, or the like. The user interface 19 is connected to the imaging processing unit 15. The user can provide various instructions to the CT imaging apparatus 10 through the user interface 19.

The X-ray generator 11 and the X-ray detector 12 are also connected to the imaging processing unit 15. The X-ray generator 11 emits an X-ray at timing and output according to a command from the imaging processing unit 15, and the X-ray detector 12 outputs a detection result to the imaging processing unit 15.

The processor 16a executes processing according to the program in the storage 17, so that the imaging unit driving mechanism 18 controls the turning operation of the turning arm 13 and controls the X-ray emission operation of the X-ray generator 11. The detection result of the X-ray detector 12 is input to the imaging processing unit 15 for each minute turning angle of the turning arm 13, and the processor 16a generates the X-ray projection image data for each minute turning angle on the basis of the detection result. The data is stored as data 17a of the X-ray projection image data group for each minute turning angle in the storage 17.

The image processing apparatus 20 includes the processing unit 30, the display 22, and the user interface 24, 26.

The processing unit 30 is connected to the display 22 and the user interface 24, 26. The processing unit 30 may receive instructions from a user via the user interface 24, 26. The processing unit 30 can control display of the display 22.

The processing unit 30 is configured by a computer including an arithmetic circuit 32 as a processor and a storage 34.

The arithmetic circuit 32 includes a processor 32a. The processor 32a may be a central processing unit (CPU). The processor 32a may include a processor for a graphics processing unit (GPU) or artificial intelligence (AI).

The storage 34 is configured by a non-volatile storage such as a flash memory or a hard disk device. The storage 34 may be a storage circuit. The storage 34 stores a program 34a and data 34b.

In the program 34a, a procedure for the image processing apparatus 20 to process image data obtained by CT imaging to generate an image for diagnosis is described.

The data 34b includes image data transmitted from the CT imaging apparatus 10, data generated by processing for generating the above-described image for diagnosis, and the like. The data 34b may be left as history data or may be erased after processing.

The processing unit 30 includes a connection port 36. The processing unit 30 is connected to the CT imaging apparatus 10 through the connection port 36. The connection port 36 may include a terminal connected to a signal line of a wired cable extending from the CT imaging apparatus 10 and a circuit for communication processing. The processing unit 30 and the CT imaging apparatus 10 may be wirelessly connected, and in this case, the connection port 36 may include a circuit for wireless processing. The data 17a of the X-ray projection image data group of the CT imaging apparatus 10 is transmitted to the processing unit 30 through the connection port 36 and stored in the storage 34.

The arithmetic circuit 32 reads the program 34a stored in the storage 34 and executes the processing described in the program 34a, so that the arithmetic circuit 32 can execute various types of processing of generating an image for diagnosis as described later. The processing unit 30 may control the CT imaging performed by the CT imaging apparatus 10.

As illustrated in FIG. 3, the processing functional unit achieved by the arithmetic circuit 32 reading the program 34a may include a biological data processing unit 33. The biological data processing unit 33 may include a biological tissue data processing unit 33a and a biological feature region data processing unit 33b.

The processing performed by the biological tissue data processing unit 33a is processing of generating three-dimensional data of the dentition constituent tissue on the basis of the image data. The dentition constituent tissue is, for example, tissue including a dentition and an alveolar bone. The dentition constituent tissue may be tissue including a dentition and a jawbone. The jawbone in the dentition constituent tissue may be a jawbone in a region supporting teeth, and may include not only the alveolar bone but also a peripheral region thereof. In the present application, the alveolar bone is a partial region of the jawbone, and is understood as a part of the jawbone that constitutes the alveolars and supports the teeth. This understanding is in line with the description of the Dental Medicine Dictionary. The dentition constituent tissue may have a horseshoe shape in plan view. Note that the plan view may be considered as a plan view in which a direction along a direction from the head side to the leg side in the axial direction of the body axis is a line-of-sight direction. The dentition constituent tissue may spread so as to have a thickness in the buccolingual direction. The dentition constituent tissue may be, for example, tissue having at least a thickness of the dentition in the buccolingual direction. The dentition constituent tissue may be tissue having a thickness of a region supporting the dentition of the jawbone. The dentition constituent tissue may include upper and lower dental arches and upper and lower jawbones supporting the upper and lower dental arches. Each of the upper and lower dental arches includes a plurality of teeth arranged in an arch shape. The upper jawbone has an alveolar bone that supports the teeth of the upper dental arch. The lower jawbone has an alveolar bone that supports the teeth of the lower dental arch. For example, the biological tissue data processing unit 33a identifies respective regions of the plurality of teeth and respective regions of the upper and lower jawbones in the three-dimensional volume data. The identification of the regions of the teeth and the upper and lower jawbones may be performed by applying a learned machine learning model. For example, as training data, a large number of pieces of data in which regions of teeth and upper and lower jawbones are mapped to the three-dimensional volume data are prepared. A machine learning model learned to segment the tooth regions and the upper and lower jawbone regions in the three-dimensional volume data is prepared using the training data. The machine learning model may be, for example, a model learned on the basis of a semantic segmentation algorithm. By applying the learned machine learning model to the three-dimensional volume data, respective regions of the plurality of teeth and respective regions of the upper and lower jawbones in the three-dimensional coordinate system are identified. As a result, the dentition region and the alveolar bone region are differentiated from each other on the basis of the three-dimensional volume data.

The identification of respective regions of the plurality of teeth and respective regions of the upper and lower jawbones in the three-dimensional volume data may be performed by region extraction processing, for example, pattern matching processing, which is regulated as a rule in advance.

The processing performed by the biological feature region data processing unit 33b is processing of detecting a characteristic region in the dentition constituent tissue on the basis of the image data. The characteristic region in the dentition constituent tissue is, for example, a region in which a lesion has occurred in the dentition constituent tissue. The lesion is a change caused by disease. The region where the lesion has occurred shows a distribution of the X-ray absorption rate different from the distribution of the X-ray absorption rate shown by the normal dentition constituent tissue. The disease is, for example, chronic pyogenic apical periodontitis, tooth root granuloma, and the like. In the case of apical periodontitis, a site having a lower X-ray absorption rate than that in the normal state and in its surrounding is generated at the peripheral edge of the tip of the tooth root. Therefore, in a case where the low X-ray absorption rate region spreads in the peripheral edge portion of the tooth root, apical periodontitis can be detected. For other lesions, a region where a lesion has occurred can be detected on the basis of three-dimensional volume data obtained by CT imaging on the basis of a position in the dentition constituent tissue such as a tooth or a jawbone, a spread pattern or a distribution pattern of an X-ray absorption rate region, and the like.

The detection by the biological feature region data processing unit 33b may be performed by applying a learned machine learning model similar to that in the identification of the dentition constituent tissue. For example, as training data, a large number of pieces of data in which regions of lesions are mapped to the three-dimensional volume data are prepared. A machine learning model learned to segment the regions of lesions in the three-dimensional volume data is prepared using the training data. By applying the learned machine learning model to the three-dimensional volume data, lesion regions in the three-dimensional coordinate system are detected.

The detection of the lesion region in the three-dimensional volume data may be performed by image extraction processing, for example, pattern matching processing.

The detection of the lesion region may not be performed on the basis of the three-dimensional volume data. For example, the lesion region may be detected by applying a machine learning model, pattern matching, or the like on the basis of slice data sliced in any direction of three-dimensional volume data.

### <Example of processing in processing unit>

### <Example of processing for display>

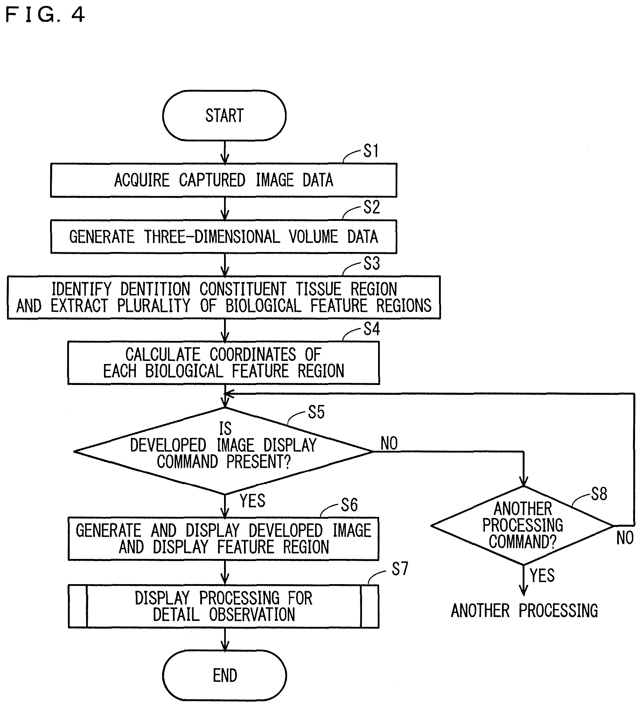

An example of data processing for display by the processing unit 30 will be described with reference to the flowchart of FIG. 4.

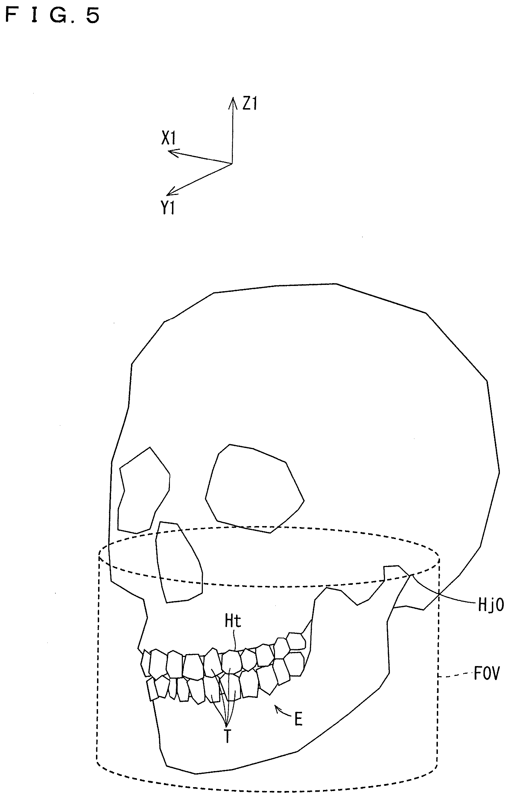

After starting the processing, in step S1, the processing unit 30 acquires the captured image data from the CT imaging apparatus 10. The captured image data is, for example, data of a field of view (FOV) region including a dental arch Ht and a jawbone Hj in a head H (See FIG. 5). For example, the FOV may be set in a cylindrical region, an elliptic columnar region, or a triangular columnar region.

In the next step S2, the processing unit 30 generates three-dimensional volume data on the basis of the captured image data.

In the present preferred embodiment, in step S1, the captured image data acquired from the CT imaging apparatus 10 is an X-ray projection image data group. Furthermore, in step S2, three-dimensional volume data is generated on the basis of the X-ray projection image data group. In the CT imaging apparatus 10, the three-dimensional volume data may be generated on the basis of the X-ray projection image data group. In this case, the processing unit 30 may acquire the three-dimensional volume data as image data.

It is also conceivable that the CT imaging apparatus 10 generates a plurality of tomographic images on the basis of the X-ray projection image data group. In this case, the processing unit 30 may generate the three-dimensional volume data by acquiring a plurality of tomographic images as the image data from the CT imaging apparatus 10 and superimposing the plurality of tomographic images.

In next step S3, the processing unit 30 executes processing of generating three-dimensional image data of the dentition constituent tissue on the basis of the image data and processing of detecting a biological feature region L in the dentition constituent tissue on the basis of the image data.

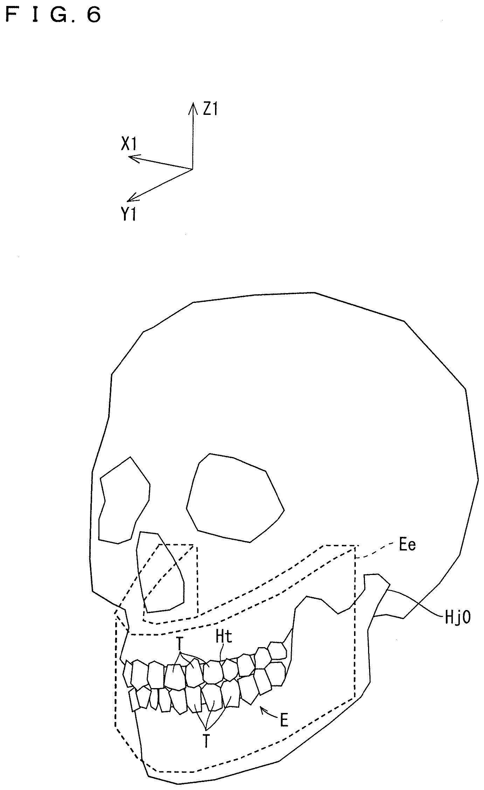

Note that, as illustrated in FIG. 6, a region Ee in which dentition constituent tissue E spreads includes the dental arch Ht in which a plurality of teeth T are arranged and the upper and lower jawbones Hj as described above. The dentition constituent tissue may include a temporomandibular joint Hjo. In the three-dimensional volume data, the region Ee in which the dentition constituent tissue E spreads may be referred to as dentition constituent tissue region Ee. The dentition constituent tissue region Ee is a horseshoeshaped region set for arithmetic operation, and may be any region that conforms to the shape and position of the dentition constituent tissue. The shape of the dentition constituent tissue region Ee may conform to the shape of the dentition constituent tissue E having the standard skeleton, and when the shape of the dentition constituent tissue E of the individual is known, the shape may be set to a shape conforming to that individual.

As described above, the processing of generating the three-dimensional image data of the dentition constituent tissue on the basis of the image data may be processing of identifying respective regions of the plurality of teeth and respective regions of the upper and lower jawbones on the basis of the three-dimensional volume data, combining the respective regions, and generating the three-dimensional data of the dentition constituent tissue.

Respective regions of the plurality of teeth and respective regions of the upper and lower jawbones may be identified by the surface layers of the teeth and jawbones, i.e. the boundaries between the interior and the exterior of the teeth and the jawbones. The plane constituted by the boundary may be considered as a surface. That is, respective regions of the plurality of teeth and respective regions of the upper and lower jawbones may be identified as the surface shape of each tooth and jawbone in the three-dimensional coordinate system of the three-dimensional volume data. Tissues of different functions constituting the living body, such as teeth and jawbones, here, tissues of different functions constituting the dentition constituent tissue of the living body may be referred to as functional dentition constituent tissue. For example, teeth are functional dentition constituent tissue having a function of biting food, and jawbones are functional dentition constituent tissue having a function of supporting teeth. The dentition constituent tissue may be considered to include a plurality of functional dentition constituent tissues. Since the region facing the teeth in the boundary of the jawbone is important, a learning model including segmentation of the alveolar inner wall may be prepared so that the alveolar inner wall can be detected with high accuracy.

In addition, the processing of detecting the biological feature region in the dentition constituent tissue on the basis of the image data may be processing of detecting a characteristic region in the dentition constituent tissue on the basis of the three-dimensional volume data as described above. In the present preferred embodiment, an example in which the biological feature region is a region where a lesion has occurred will be described. Here, in a case where the biological feature regions are, for example, a plurality of lesion regions, naturally, the assembly of the biological feature regions is constituted by collecting individual lesion regions. As this individual lesion region, an individual biological feature region constituting the assembly of biological feature regions may be referred to as unital biological feature region. A region where a plurality of unital biological feature regions are collected may be referred to as collective biological feature region. The image of the biological feature region may be referred to as biological feature region image.

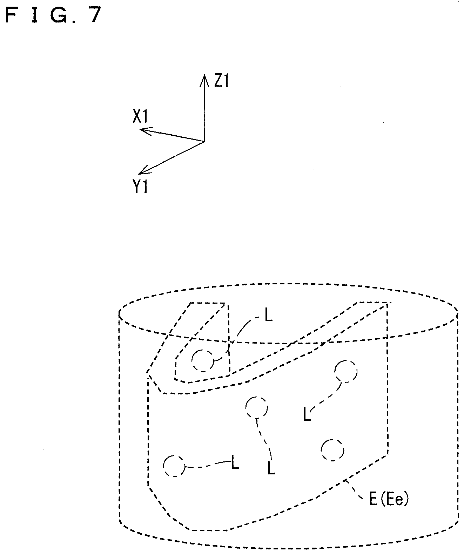

By step S3, three-dimensional image data of the dentition constituent tissue E including the dentition and the jawbone is generated in the FOV, and the presence of lesion regions L (biological feature regions L) with respect to the dentition constituent tissue E is detected (See FIG. 7).

The processing unit 30 can detect a plurality of the biological feature regions. That is, in a case where one biological feature region (unital biological feature region) is detected, the processing unit 30 continues the detection of other biological feature regions without ending the detection of the biological feature regions. Thus, in a case where a plurality of biological feature regions are present in the dentition constituent tissue E, the plurality of biological feature regions (collective biological feature region) are detected.

Note that, in the present preferred embodiment, description will be made on the premise that some kind of biological feature region is present. In a case where no biological feature region is detected, the processing may end. Furthermore, in a case where the number of detected biological feature regions is one, the processing of changing focusing described later may be omitted.

In step S4, the processing unit 30 calculates a three-dimensional position, that is, three-dimensional coordinates, at which the detected biological feature region is present in the coordinate system of the three-dimensional image data of the dentition constituent tissue E. As the three-dimensional coordinates of the three-dimensional image data, in arithmetic operation, on the basis of the spatial coordinates of the CT imaging apparatus, for example, on the basis of the orientation of the subject at the time of imaging, an X1 direction which is the same direction as the X direction, a Y1 direction which is the same direction as the Y direction, and a Z1 direction which is the same direction as the Z direction are set. The three-dimensional position at which the biological feature region is present may be referred to as biological feature region three-dimensional position.

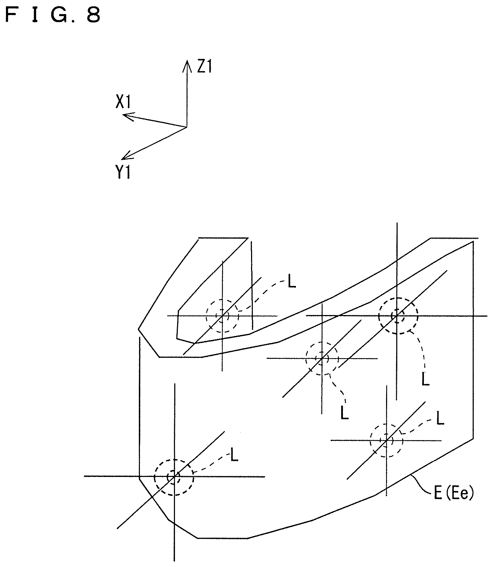

The coordinates of the biological feature region are a position to be displayed as a position at which the biological feature region is present in a developed image described below. The coordinates of the biological feature region may be point coordinates located within the boundary of the lesion region detected in step S4. The coordinates of the biological feature region may be, for example, the geometric center of the surface of the lesion region detected in step S3 (See FIG. 8). It may be any position (for example, a lower end or an upper end) on the surface of the lesion region.

In next step S5, the processing unit 30 determines the presence or absence of the display command of the developed image. The display command of the developed image is received using the user interface 24, 26. When the user inputs the display command of the developed image using the user interface 24, 26, the processing unit 30 determines that the display command of the developed image is present, and the processing proceeds to step S6. In a case where the user does not input the display command of the developed image, the processing unit 30 determines that the display command of the developed image is absent, and the processing proceeds to step S8.

In step S8, the processing unit 30 determines the presence or absence of another command via the user interface 24, 26, and the like. When it is determined that another command is present, the processing unit 30 executes the corresponding other processing. When it is determined that another command is absent, the processing returns to step S5, and the processing unit 30 repeats the processing of determining the presence or absence of the display command of the developed image.

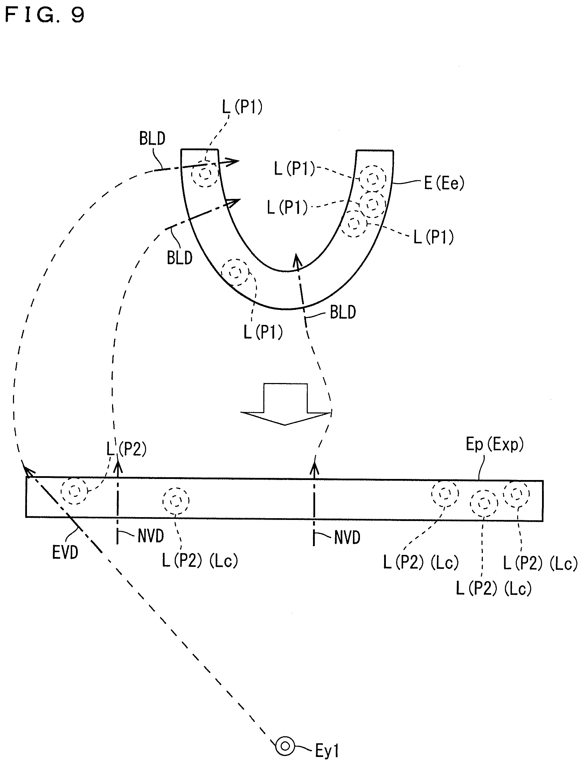

In a case where the processing proceeds to step S6, the processing unit 30 generates a dentition developed image Ep in which the dentition constituent tissue E is developed on the basis of the three-dimensional image data of the dentition constituent tissue E. The dentition developed image Ep is an image in which the dentition constituent tissue E having an arch shape or a horseshoe shape in plan view is developed so as to form a straight line in plan view (See FIG. 9). For example, the dentition developed image Ep is generated as follows. That is, at each coordinate position in the up-down direction, a curve having an arch shape or a horseshoe shape passing through the center in the buccolingual direction of the dentition constituent tissue E is calculated. At each position along the curve, the presence or absence or transmittance of an image in a direction orthogonal to a tangent of the curve is calculated. The presence or absence or transmittance of an image at respective coordinates on the curve is expressed as presence or absence or transmittance of an image at respective linear coordinates. At respective coordinate positions in the up-down direction, the above processing is performed and data of the processing is overlapped in the up-down direction, whereby the dentition developed image Ep is generated.

The generation of the dentition developed image Ep may be performed by development of the dentition constituent tissue E or by development of the region Ee, in which the dentition constituent tissue E spreads. The development used herein may be regarded as making a large curvature smaller in plan view. The curvature may be the curvature of a line passing through the center in the buccolingual direction of the dentition constituent tissue E or the region Ee. The development may be regarded as making such a large curvature smaller when seen in the Z1 direction. Making a large curvature smaller may include changing a curved state to a flat state, more specifically, may include changing the dentition constituent tissue E or the region Ee from a curved state to a flat state in plan view. A state with a small curvature may include a case in which the curvature is zero.

Note that, in a case where the three-dimensional image data of the dentition constituent tissue E is expressed by the translucent data of the surface of the dentition constituent tissue E, the boundary portion of the dentition constituent tissue in the dentition developed image Ep is expressed in a dark color with low transmittance.

The dentition developed image Ep is a kind of panoramic image in which the dental arch is planarly developed. The dentition developed image Ep may also extend to the alveolar bone and the jawbone. Furthermore, the dentition developed image Ep may also extend to the jawbone. The dentition developed image Ep may be an image in which the dentition constituent tissue E is passed through in the thickness direction thereof, or may be a tomographic image at an arbitrary position in the thickness direction.

The dentition developed image Ep is preferably an image in which the entire region of the dentition constituent tissue E is viewed frontally. As illustrated in FIG. 9, the image in which the entire region of the dentition constituent tissue E is viewed frontally is, assuming that the dentition developed image Ep is developed in the direction orthogonal to a median line Md of the head H, an image in which the entire dentition constituent tissue E is viewed from the front of the median line of the head H (from a frontally-viewing direction Dv in which the front teeth region is viewed frontally). The image data of the dentition developed image Ep has a thickness of the dentition constituent tissue E. The image data of the dentition developed image Ep has at least a thickness of dental arches in the buccolingual direction.

Not only the three-dimensional image data of the dentition constituent tissue E but also data obtained by converting the three-dimensional volume data into a panoramic image may be superimposed on the dentition developed image Ep. The dentition developed image Ep does not necessarily develop so as to form a linear shape in plan view, and may include curvature or bending. That is, the dentition developed image Ep may be an image in which the entire dentition constituent tissue E can be observed at a glance. For example, the development may be performed as processing such that the amount of forward displacement becomes larger in the regions on the right and left end sides of the dentition constituent tissue region Ee than in the region near the center. When linear development or substantially linear development in plan view is expressed as "being developed flatly", the dentition developed image Ep may be formed so as to become a flatly developed image. Image processing for generating the dentition developed image Ep so that the entire dentition constituent tissue E can be observed at a glance may be referred to as development processing. In particular, the development processing of flatly developing may be referred to as flat development processing. The development of flatly developing may be referred to as flat development.

By similarly applying the above processing to the biological feature region, a biological feature region corresponding position P2 in the dentition developed image Ep is calculated. The biological feature region corresponding position P2 may be regarded as a position obtained by converting the three-dimensional position P1 of the biological feature region calculated in step S4 by the same processing as the geometric conversion processing of developing the dentition constituent tissue E in the dentition developed image Ep.

The three-dimensional position P1 can be defined, for example, at the center of the biological feature region. The center of the biological feature region may be a geometric center or a centroid. As the arithmetic operation target, the three-dimensional position P1 may be the position of a spot (that is, a position indicating a specific point) as described above, but, in a case where the biological feature region has a spread, may be the position of at least a part of the region. It is conceivable that the three-dimensional position P1 is, for example, a certain region around the center including the center. The three-dimensional position may be a position of the entire biological feature region. Therefore, the corresponding position P2 may also be a case of the position of the spot or a case of the position of the region as the arithmetic operation target.

A region set in arithmetic operation in which the dentition constituent tissue region Ee is developed may be considered as a dentition developed region Ex. Also for the dentition constituent tissue region Ee, the development of flatly developing may be referred to as flat development, and in particular, the dentition developed region Ex in which flat development is performed may be considered as a dentition flatly-developed region Exp.

The development of the dentition constituent tissue E may be performed by development processing of developing the image data of the dentition constituent tissue E in the dentition constituent tissue region Ee so as to conform to the shape of the dentition developed region Ex. Hereinafter, it will be described that the region occupied by the dentition constituent tissue E and the dentition constituent tissue region Ee coincide with each other, and the region occupied by the dentition developed image Ep and the dentition developed region Ex coincide with each other.

The state of the dentition constituent tissue region Ee before development may be referred to as pre-development curved state, and the state of the dentition developed region Ex after development may be referred to as post-development at-a-glance state. It is preferable that the development processing is performed such that a buccolingual direction BLD in the pre-development curved state corresponds to a frontally-viewing direction NVD in the post-development at-a-glance state.

The frontally-viewing direction may be a direction orthogonal to the frontally-viewed surface of the dentition developed region Ex or a normal direction, but a line-of-sight direction based on another idea may be set. For example, a virtual viewpoint Ey1 may be set on the center of the dentition developed region Ex in the frontally-viewing line-of-sight direction to perform image processing along a line-of-sight direction EVD from this viewpoint.

In the dentition developed region Ex and/or the dentition developed image Ep, a region located at the biological feature region corresponding position P2 and corresponding to the biological feature region L may be referred to as biological feature region corresponding region Lc.

Since the dentition constituent tissue region Ee has a thickness in the buccolingual direction, the dentition developed region Ex also has a thickness in the frontally-viewing direction corresponding to the thickness in the buccolingual direction. Furthermore, since the dentition constituent tissue E has the above-described thickness in the buccolingual direction, the dentition developed image Ep also has a thickness in the frontally-viewing direction corresponding to the buccolingual direction.

Since the dentition developed region Ex has a thickness corresponding to the dentition constituent tissue region Ee, unlike the thin panoramic tomograph, even if there is a deviation in the buccolingual direction at the position where the biological feature region is present, the dentition developed region Ex can be accommodated in the region (in the image processing, the biological feature region image is accommodated in the region without blurring).

The coordinates of the three-dimensional position P1 may be calculated as coordinates in the FOV region or as coordinates in the dentition constituent tissue region Ee.

In the development, arithmetic operation for associating the three-dimensional position P1 of the biological feature region with the biological feature region corresponding position P2 may be performed. The arithmetic operation for performing this association may be referred to as original coordinates arithmetic operation. In the original coordinates arithmetic operation, arithmetic operation of identifying which position in the buccolingual direction the three-dimensional position P1 of the biological feature region is located may be performed. In addition, arithmetic operation of identifying which position in the frontally-viewing direction corresponding to the buccolingual direction the biological feature region corresponding position P2 is located may be performed.

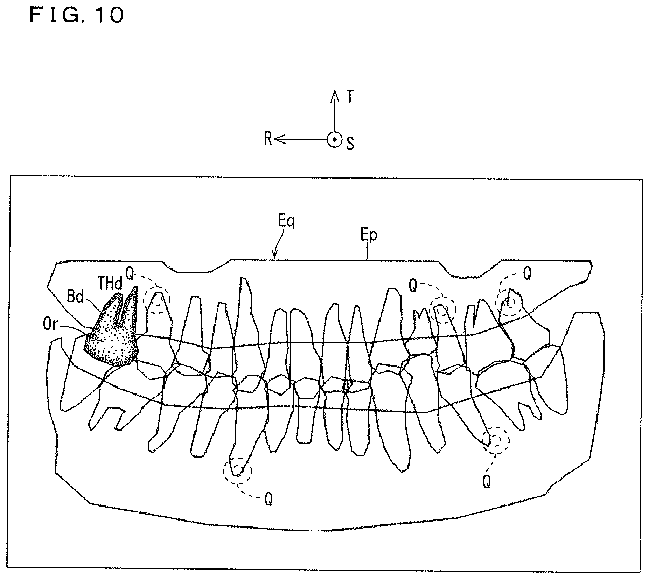

Then, as illustrated in FIG. 10, the processing unit 30 displays, on the display 22, a biological feature region arrangement developed image Eq in which annotation images Q are superimposed on the dentition developed image Ep.

For arithmetic operation, three-dimensional coordinates may be set in the dentition developed region Ex. For example, the right-left extending direction due to the development of the dentition developed region Ex is defined as an R direction. A direction parallel to the body axis is defined as a T direction. In the present preferred embodiment, the T direction, the Z direction, and the Z1 direction are the same direction. A direction parallel to the frontally-viewing direction is defined as an S direction. In the dentition developed image Ep, the right-left direction is defined as the R direction, and the up-down direction is defined as the T direction. When conditions for identifying RT coordinates on the dentition developed image Ep and for identifying the S direction are determined, mutual identification with X1Y1Z1 coordinates is enabled. Therefore, like the biological feature region corresponding region Lc in the dentition developed region Ex, the region in which S coordinates are determined together with the RT coordinates can be mutually identified with the X1Y1Z1 coordinates of the biological feature region L. The coordinates on the three-dimensional image data such as the X1Y1Z1 coordinates may be referred to as three-dimensional image data coordinates, and the three-dimensional coordinates may be referred to as three-dimensional image data three-dimensional coordinates. The coordinates on the dentition developed region Ex such as RST coordinates may be referred to as dentition developed region coordinates, and the three-dimensional coordinates may be referred to as dentition developed region three-dimensional coordinates.

The annotation image Q is an image indicating a biological feature region. The annotation image Q may be an image exhibiting a color, a shape, or a change that enables differentiation from the dentition developed image Ep spreading in the background. For example, the annotation image Q may be an image exhibiting a hue different from that of the dentition developed image Ep. More specifically, the annotation image Q may be an image exhibiting a warm color that is easily noticeable, for example, red, orange, or yellow. The annotation image Q may be an image with a display change that enables differentiation from the dentition developed image Ep, for example, blinking, a color change, or a shape change. The annotation image Q may have a shape that enables differentiation from the dentition developed image Ep, for example, a circle, a regular polygon, a radiation shape, a cross shape, an exclamation mark shape, or the like.

The annotation image Q may have a shape indicating the outline of the lesion region L. In this case, it is possible to know the approximate shape and size of the lesion region L by viewing the biological feature region arrangement developed image Eq.

At least one annotation image Q is superimposed on the dentition developed image Ep so as to be located at the above-described biological feature region corresponding position P2 in the dentition developed image Ep.

In the example illustrated in FIG. 10, the boundary between the tooth and the jawbone is indicated by a line in the dentition developed image Ep. In addition, the annotation image Q is indicated by a double circle forming a broken line. In the example illustrated in FIG. 10, five annotation images Q are illustrated. That is, in a case where a plurality of biological feature regions are detected, a plurality of annotation images Q respectively corresponding to the plurality of biological feature regions are displayed in a recognizable manner in the biological feature region arrangement developed image Eq. For example, as in a tooth image THd illustrated in FIG. 10, translucent processing of the functional dentition constituent tissue may be performed such that the boundary of the functional dentition constituent tissue (which may be considered as the boundary between the presence and the absence) is constituted by dots having uniform and sparse density. As a result, a surface region Or orthogonal to the line-of-sight direction has high transparency, and a surface region Bd along the line-of-sight direction has low transparency, so that the boundary can be easily visually recognized while being translucent. Note that the dots may be non-uniform to such an extent that difficulty in visual recognition is not caused. Since it is sufficient that the boundary surface has transparency, the image processing is not limited to dots, and may be image processing of a see-through surface such as a mesh. In both the dot and the mesh, the sparseness and denseness vary depending on the line-of-sight direction, so that the transparency varies. Image processing in which the transparency changes depending on the line-of-sight direction may be referred to as line-of-sight direction-corresponding transparency image processing.

An element that increases transparency, such as dots in a sparse state as compared with a dense state or eliminated dots, is referred to as pro-transparency element. Furthermore, increasing the transparency is referred to as pro-transparency.

An element that lowers the transparency, such as dots in a dense state as compared with the sparse state or nonexistent state, is referred to as anti-transparency element. Furthermore, lowering the transparency is referred to as anti-transparency.

The line-of-sight direction-corresponding transparency image processing may be a combination of at least any one of the following group A and at least any one of the following group B.
Group A:
   ▪ Processing of enlarging or increasing the pro-transparency element of the frontally-viewed boundary surface
   ▪ Processing of promoting pro-transparency of the frontally-viewed boundary surface
   ▪ Processing of downsizing, or reducing or eliminating the anti-transparency element of the frontally-viewed boundary surface
   ▪ Processing of suppressing anti-transparency of the frontally-viewed boundary surface
Group B:
   ▪ Processing of downsizing, or reducing or eliminating the pro-transparency element of the obliquely-viewed or laterally-viewed boundary surface
   ▪ Processing of suppressing pro-transparency of the obliquely-viewed or laterally-viewed boundary surface
   ▪ Processing of enlarging or increasing the anti-transparency element of the obliquely-viewed or laterally-viewed boundary surface
   ▪ Processing of promoting anti-transparency of the obliquely-viewed or laterally-viewed boundary surface
   The line-of-sight direction-corresponding transparency image processing may also be performed on the alveolar bone and the jawbone part.

In the present preferred embodiment, in order to describe change processing of focusing described later, description is made on the premise that a plurality of biological feature regions are present. Note that, even in a case where a plurality of biological feature regions are present, it is also conceivable that one or a plurality of annotation images Q corresponding to some of the plurality of biological feature regions are displayed.

In a case where the biological feature region is not detected in step S3, the processing is not ended and the dentition developed image Ep in which the annotation image Q is not present may be displayed.

Step S5 may be omitted, and after the processing of step S4 is ended, the processing of step S6 may be performed regardless of the presence or absence of a command.

After step S6, in step S7, display processing for detail observation is performed. When this display processing ends, the processing of the processing unit 30 ends.

### <Focus processing>

The processing of step S7 includes processing of generating a detail observation image, processing of determining a focus order, and processing of subjecting biological feature regions to focusing in the focus order and displaying the detail observation image on the display 22. As a result, in the biological feature region arrangement developed image Eq, the biological feature regions are subjected to focusing in the focus order, and a detail observation image of the focused biological feature region is displayed. Therefore, the user can observe the detail observation image while recognizing the position of the focused biological feature region by viewing the biological feature region arrangement developed image Eq. Since the biological feature regions are subjected to focusing in a predetermined focus order, it is easy to observe the biological feature regions without omission and without duplication. The focus order of the biological feature regions may be the order of focusing from one unital biological feature region to another unital biological feature region. Targets for determining the focus order are the same kind of collective biological feature regions such as "lesions" and "root apexes", for example, and the focusing targets are different when the targets of interest are different. Such the same kind of biological feature regions to be the targets of interest may be referred to as "kind-of-interest biological feature region".

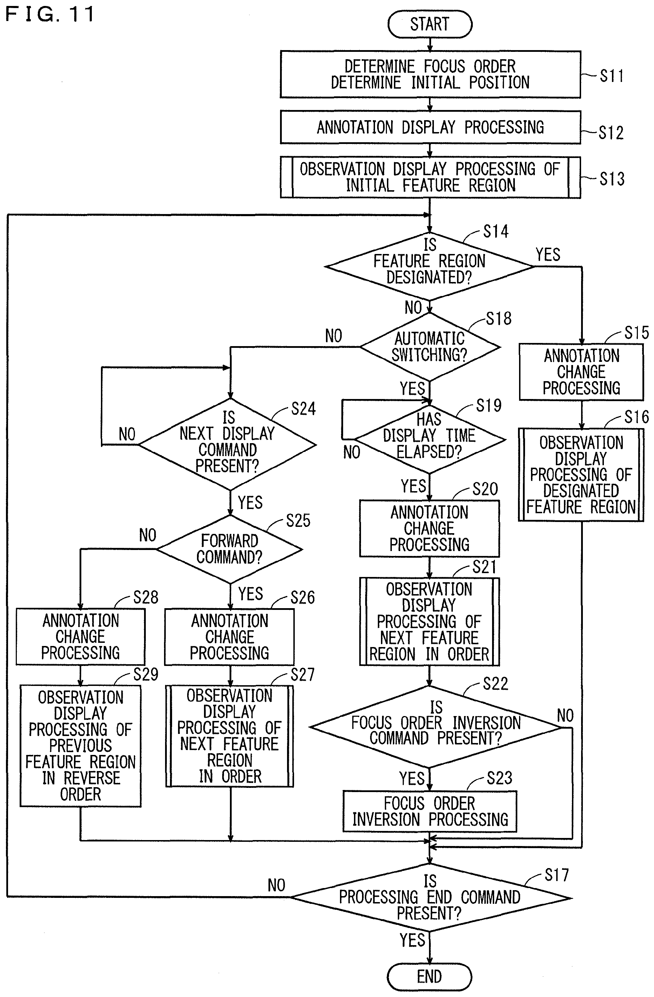

The processing of step S7 will be described more specifically with reference to the flowchart illustrated in FIG. 11.

In step S11, the processing unit 30 determines the focus order. The focus order is determined according to a rule predetermined by the program 34a. The rule may be a rule for determining the focus order on the basis of the plurality of biological feature region corresponding positions P2 in the dentition developed image Ep. For example, the rule may be defined by a rule that the biological feature region corresponding positions P2 located in the upper dentition and jawbone are ranked ahead of the biological feature region corresponding positions P2 located in the lower dentition and jawbone, and a rule that display is performed in order from one side to the other side in the right-left direction (for example, sequentially displayed from left to right in front view). In the present preferred embodiment, the following description will be given assuming that the rules are applied.

Note that the rule for determining the focus order may be another rule. For example, the rule may be defined only by a rule that display is performed in the order from one side to the other side in the right-left direction (for example, sequentially displaying from left to right in front view).

By determining the focus order, for example, focus order data in which a plurality of biological feature region corresponding positions P2 are stored in a predetermined array is generated.

In addition, the processing unit 30 determines an initial position. The initial position may be, for example, a first position in the determined focus order. For example, the biological feature region corresponding position P2 located at the leftmost position in the upper dentition and jawbone may be set as the initial position. The feature region corresponding to the initial position may be referred to as initial feature region.

Next, in step S12, annotation display processing is performed. The annotation display processing is processing of displaying the annotation image Q in the biological feature region arrangement developed image Eq so that the position of the feature region displayed as the detail observation image D can be recognized in the dentition developed image Ep. In step S12, the detail observation image D corresponding to the initial feature region is displayed. Therefore, for example, the annotation image Q corresponding to the initial feature region is displayed so as to be differentiated from other annotation images Q. An example of displaying any one of the annotation images Q so as to be differentiated from other annotation images Q will be described in step S20.

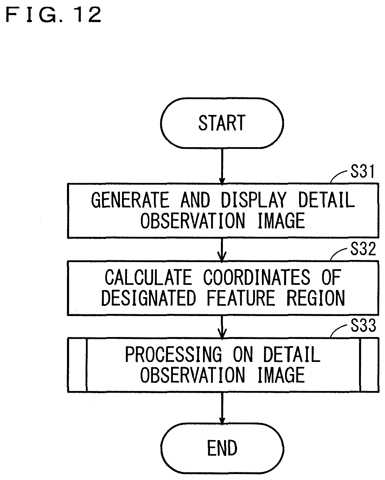

In next step S13, processing of displaying a feature region corresponding to the initial position for observation is performed. Here, the processing of step S13 will be described more specifically with reference to the flowchart in FIG. 12.

In step S13, processing of generating and displaying the detail observation image suitable for observation is executed.

That is, in step S31, the three-dimensional coordinates of the initial feature region identified in step S11 are calculated. For example, in step S6, when the dentition developed image Ep in which the dentition constituent tissue E is developed is generated on the basis of the three-dimensional image data of the dentition constituent tissue E, the three-dimensional position P1 of the biological feature region is converted into the biological feature region corresponding position P2. The correspondence therebetween is stored in the storage 34 as a table. With reference to the table, the three-dimensional coordinates of the initial feature region are calculated. Alternatively, the three-dimensional coordinates of the initial feature region may be calculated by inverse conversion processing of geometric conversion processing of developing the dentition constituent tissue E in the dentition developed image Ep. As a result, the position of the initial feature region in the three-dimensional image data of the dentition constituent tissue E is identified.

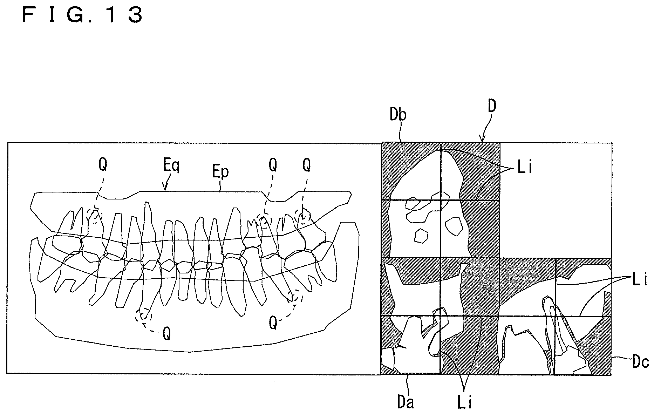

In next step S32, the detail observation image D at the three-dimensional position of the three-dimensional image data is generated on the basis of the three-dimensional image data of the dentition constituent tissue E. Then, the detail observation image D is displayed on the display 22 simultaneously with the biological feature region arrangement developed image Eq (See FIG. 13). Note that the detail observation images D at the plurality of three-dimensional positions may be generated before display, or may be collectively generated at the time of detection of the plurality of biological feature regions.

The detail observation image D is an image that can be observed in more detail than the dentition developed image Ep and is suitable for more detailed diagnosis. In the present preferred embodiment, an example in which the detail observation image D includes a plurality of cross-sectional images Da, Db, Dc, more specifically, three cross-sectional images Da, Db, Dc orthogonal to each other will be described (See FIG. 13).

The three cross-sectional images Da, Db, Dc are cross sections in planes passing through the three-dimensional position P1 of the biological feature region and orthogonal to each other. In the three-dimensional image data of the dentition constituent tissue E, the plane passing through the three-dimensional position P1 is identified, and the distribution of the X-ray transmittance along the plane is obtained, whereby the three cross-sectional images Da, Db, Dc are generated. The three cross-sectional images Da, Db, Dc may be distribution of X-ray transmittance in a thick slice layer. Since the correspondence between the three-dimensional position P1 of the biological feature region and the biological feature region corresponding position P2 is identified in the arithmetic operation of the development processing, which of the coordinates of the FOV region the coordinates of the three-dimensional position P1 correspond to and/or which of the coordinates of the dentition constituent tissue region Ee the coordinates correspond to is also identified. The same applies to the coordinates of each spot in the region indicated by the dentition developed image Ep. Of course, the original coordinates arithmetic operation described above may be performed.

The three cross-sectional images Da, Db, Dc may include the cross-sectional image Dc orthogonal to the buccolingual direction, the cross-sectional image Db orthogonal to the up-down direction, and the cross-sectional image Da orthogonal to both the cross-sectional images Dc, Db. The cross-sectional image Dc orthogonal to the buccolingual direction may be a cross section along the tangential direction of the curve along the extending direction of the dentition constituent tissue E. The cross-sectional image Dc orthogonal to the buccolingual direction is an example of a cross-sectional image in which the biological feature region is viewed frontally. Since what is assigned to which cross-sectional image is arbitrarily determined as the default cross-section, it is possible to appropriately change, among the cross-sectional image Da, the cross-sectional image Db, and the cross-sectional image Dc, which of the cross-sectional image orthogonal to the buccolingual direction, the cross-sectional image orthogonal to the up-down body axis direction, and the cross-sectional image orthogonal to both of these cross-sectional images is to be assigned.

The detail observation image D may be, for example, a cross-sectional image having a higher resolution than the dentition developed image Ep or a transparent image. The detail observation image D may be a three-dimensional image in which the line-of-sight direction can be changed.

As a result, the detail observation image D at the three-dimensional position P1 of the biological feature region corresponding to the annotation image Q selected through the user interface 24, 26 is generated.

In the display 22, the detail observation image D is displayed on the display 22 simultaneously with the biological feature region arrangement developed image Eq. At this time, the position of the detail observation image D with respect to the biological feature region arrangement developed image Eq is arbitrary. In FIG. 13, the biological feature region arrangement developed image Eq and the detail observation image D are disposed laterally side by side. The biological feature region arrangement developed image Eq and the detail observation image D may be disposed vertically side by side. The detail observation image D may partially enter the biological feature region arrangement developed image Eq and be displayed in a superimposed manner.

In addition, the layout of the cross-sectional images Da, Db, Dc in the detail observation image D is arbitrary. In FIG. 13, the square display region of the detail observation image D is divided into two upward and downward and divided into two right and left. The cross-sectional image Dc orthogonal to the buccolingual direction is displayed in the lower right region, the cross-sectional image Db orthogonal to the body axis direction is displayed in the upper left region, and the cross-sectional image Da orthogonal thereto is displayed in the lower left region.

Each of the three cross-sectional images Da, Db, Dc may include a reference mark Li indicating positions of the other cross sections. The reference mark Li may be a straight line, a broken line, a short line located in the central region of the cross-sectional images Da, Db, Dc, or two marks located in the peripheral edges of the cross-sectional images Da, Db, Dc. That is, the reference mark Li may be any display that can indicate linear positions as positions of other cross sections. In FIG. 13, a vertical straight line and a horizontal straight line indicating positions of the other cross sections are illustrated as the reference mark Li in each of the cross-sectional images Da, Db, Dc.

In next step S33, display processing on the detail observation image D may be performed. After the display processing ends, the processing of step S13 may end. Specifically, step S33 is processing mainly including step S40, which is a start step of detail observation image adjustment operation-related processing described later, and step S43, which is a step of generating and displaying the detail observation image after adjustment.

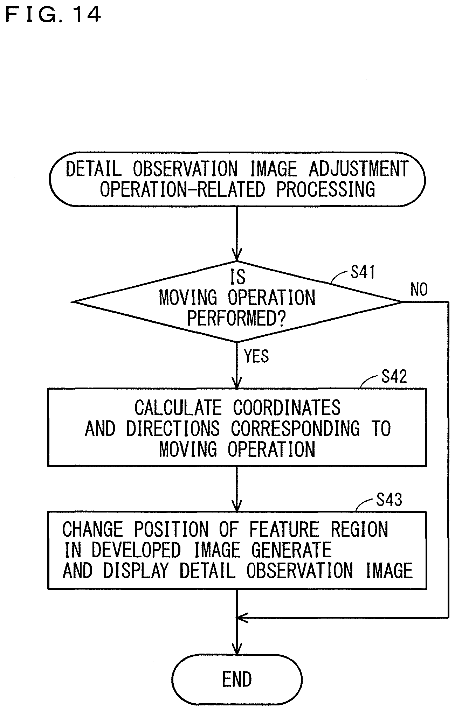

The display processing on the detail observation image D may be, for example, processing of changing the cross-sectional positions of the cross-sectional images Da, Db, Dc as illustrated in the flowchart of FIG. 14.

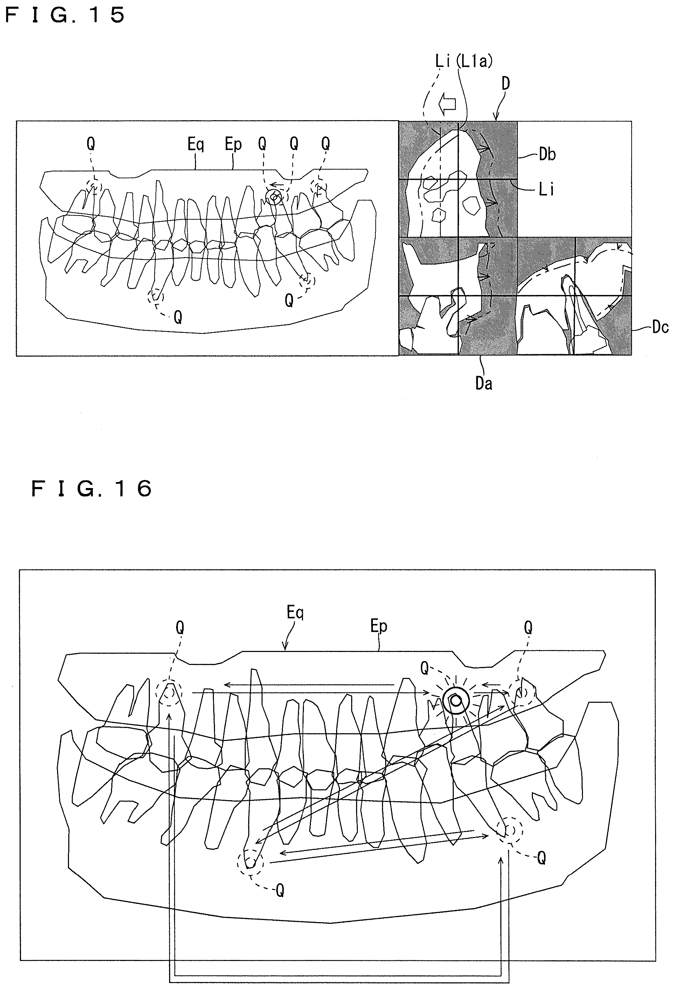

That is, after the detail observation image D is displayed, the processing proceeds to step S40, the detail observation image adjustment operation-related processing is started, and step S41 is processed. In step S41, the processing unit 30 determines whether the moving operation is performed. The moving operation is received, for example, by an operation on the reference mark Li through the user interface 24, 26. For example, as illustrated in FIG. 15, by moving any reference mark Li (referred to as reference mark Lia in FIG. 15) with a pointer device such as a mouse, the cross-sectional position indicated by the reference mark Lia is moved. As a result, the moving operation is received. The moving operation may be performed by a switch device such as a keyboard.

The moving operation in step S41 may be an operation of changing the inclination of the reference mark Li. For example, in a case where the central region of the reference mark Li is subjected to a drag operation by a pointer device such as a mouse, the position of the reference mark Li may be changed, and, in a case where the end region of the reference mark Li is subjected to the drag operation by a pointer device such as a mouse, the inclination of the reference mark Li may be changed. The rotation operation of the three-dimensional image may be performed by applying a point operation to a portion deviating from the reference mark Li of the cross-sectional image Da, the cross-sectional image Db, and the cross-sectional image Dc. The inclination of the reference mark Li is changed according to the direction and amount of the drag operation, and a moving operation of inclining the cross-sectional position is received.

When it is determined in step S41 that the moving operation is not performed, the display processing on the detail observation image D (step S33) is ended, and when it is determined that the moving operation is performed, the processing proceeds to step S42.

In step S42, coordinates and a direction corresponding to the moving operation are calculated. The coordinates corresponding to the moving operation are coordinates obtained by shifting the coordinates of the three-dimensional position P1 before the movement by the movement amount corresponding to the moving operation. By converting the coordinates after the moving operation by processing similar to the processing of converting the three-dimensional position P1 into the biological feature region corresponding position P2, the coordinates of the biological feature region after the moving operation in the biological feature region arrangement developed image Eq are calculated. In addition, the direction corresponding to the moving operation is a direction obtained by inclining a direction orthogonal to any of the cross-sectional images Da, Db, Dc at the three-dimensional position P1 before the movement by an inclination corresponding to the moving operation.

In next step S43, the processing unit 30 changes the position of the annotation image Q corresponding to the initial position in the dentition developed image Ep to the position after the moving operation. Furthermore, the cross-sectional images Da, Db, Dc are generated and displayed using the coordinates and the direction after the moving operation as references. After the moving operation, the cross-sectional images Da, Db, Dc are generated so as to be disposed around the coordinates after the moving operation as a center. Therefore, in the cross-sectional images Da, Db, Dc, the image before the operation is displayed with being shifted to the opposite side to the moving operation direction, or the image in the depth or front direction of the image before the operation is displayed (See the outline indicated by the two-dot chain line in FIG. 15).

Thereafter, the display processing (step S43) on the detail observation image D is ended.

In a case where the moving operation is performed again, the processing after step S41 may be repeated.

In this manner, the detail observation image D is displayed according to the feature region determined as the initial position, and the cross-sectional position can be adjusted with respect to each of the cross-sectional images Da, Db, Dc.

The processing in FIG. 12 and FIG. 14 is processing performed not only on the feature region determined as the initial position but also on the feature region designated by the user and the feature region determined according to a predetermined focus order. As illustrated in FIG. 14, the processing of changing the cross-sectional position may be omitted.

The description returns to the description based on the flowchart illustrated in FIG. 11.

When the processing of step S13 ends, the processing proceeds to step S14.

In step S14, the processing unit 30 determines whether the feature region is designated. The designation of the feature region may be performed, for example, by the user selecting any one of the plurality of annotation images Q with a pointer device such as a mouse. The designation of the feature region may be performed by designating the annotation image Q with a switch device such as a keyboard. When it is determined that the feature region has been designated, the processing proceeds to step S15, and when it is determined that the feature region is not designated, the processing proceeds to step S18.

In step S15, annotation change processing is performed. The annotation change processing is processing of changing the annotation image Q in the biological feature region arrangement developed image Eq so that the position of the feature region displayed as the detail observation image D can be recognized in the dentition developed image Ep. In step S15, the detail observation image D corresponding to the designated feature region is displayed. Therefore, for example, the annotation image Q corresponding to the designated feature region is displayed so as to be differentiated from other annotation images Q. This display example may be similar to that in step S20 described later.

In next step S16, processing of displaying the designated feature region for observation is performed. This processing is performed, for example, by executing the processing illustrated in FIG. 12 and FIG. 14 on the designated feature region similarly to step S13. As a result, the detail observation image D is displayed for the designated feature region. After the processing ends, the processing proceeds to step S17.

In step S17, it is determined whether the processing for diagnosis is ended. When it is input through the user interface 24, 26 or the like that the processing is ended, the processing is ended. In a case where the processing is not ended, the processing returns to step S14 and the subsequent processing is repeated.

When it is determined in step S14 that the feature region is not designated, the processing in and after step S18 is executed for executing the processing described below. The processing is executed, when displaying the biological feature region arrangement developed image Eq, to indicate that the biological feature regions are subjected to focusing in the focus order by a change in the annotation images Q and to display, on the display 22, the detail observation images D corresponding to focusing of the biological feature regions.

In step S18, it is determined whether to automatically switch the focus target. Whether to automatically switch the focus target is received, for example, by an operation through the user interface 24, 26. For example, in the initial state, the mode may be set to the automatic switching mode, and, in a case where any key is operated, the mode may be switched to the manual switching mode other than the automatic switching. When it is determined that the mode is the automatic switching mode, the processing proceeds to step S19, and when it is determined that the mode is not the automatic switching mode, the processing proceeds to step S24.

When the processing proceeds to step S19, it is determined whether the display time has passed a predetermined time. The display time may be the display time of the annotation image Q corresponding to the initial position or the display time of the detail observation image D. The predetermined time is an arbitrarily determined time, and is, for example, a time suitable for detail observation by the detail observation image D. The processing of step S19 is repeated until the display time elapses a predetermined time, and when it is determined that the display time elapses a predetermined time, the processing proceeds to step S20.

In step S20, the processing unit 30 identifies, according to the focus order, the feature region that comes next in the order after the feature region being displayed, and alters the annotation image Q so that the next feature region in the order can be differentiated from other feature regions as illustrated in FIG. 16.

For example, as illustrated in FIG. 16, a plurality of annotation images Q are superimposed on a biological feature region arrangement developed image Eq, and some annotation images targeted for focusing among the plurality of annotation images Q may be displayed in a different manner from the rest of annotation images Q.

For displaying in a different manner, for example, the annotation image Q corresponding to the next feature region in the order may be displayed brighter than other annotation images Q.

The annotation image Q corresponding to the next feature region in the order may be displayed at a blinking speed different from that of other annotation images Q. Here, the blinking speed is the number of times of blinking in a unit time. The blinking speed of 0 times/hour means that no blinking occurs. Examples of the case where the blinking speed is different include a case where there is a difference between a case where no blinking occurs (that is, the case where lighting is continued) and a case where the blinking occurs. For example, the annotation image Q corresponding to the next feature region in the order may blink, and other annotation images Q may be displayed in a lighted manner. In addition, the annotation image Q corresponding to the next feature region in the order may blink, and other annotation images Q may also blink at a blinking speed different from that of the annotation image Q corresponding to the next feature region in the order.

The annotation image Q corresponding to the next feature region in the order may be displayed in a color different from that of other annotation images Q. For example, the annotation image Q corresponding to the next feature region in the order may be displayed in red, and other annotation images Q may be displayed in orange.

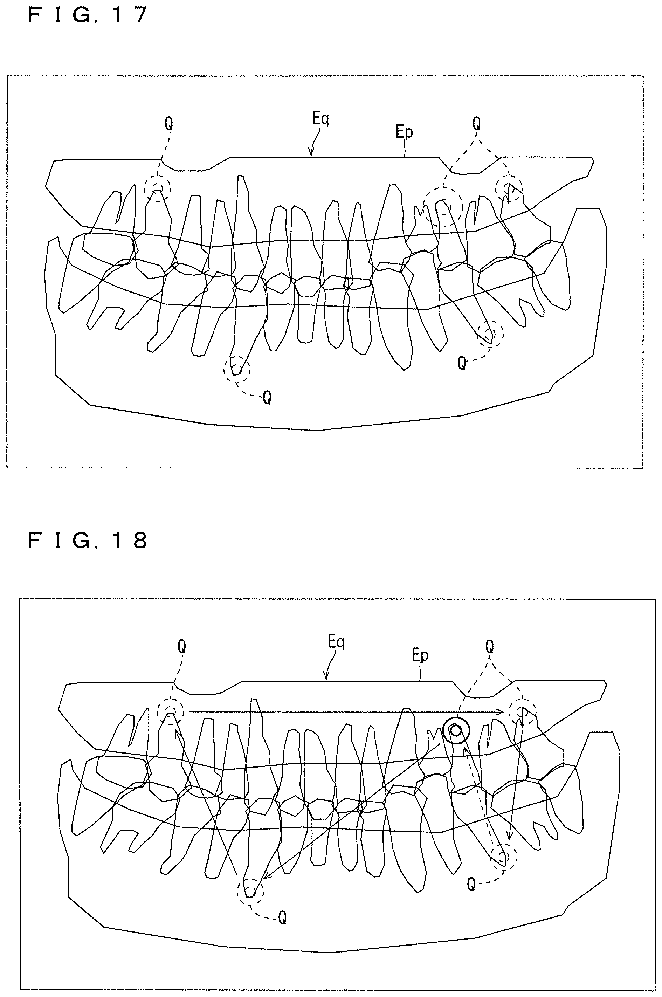

The annotation image Q corresponding to the next feature region in the order may be displayed in a size different from that of other annotation images Q. For example, the annotation image Q corresponding to the next feature region in the order may be displayed larger than other annotation images Q (See FIG. 17).

As described above, a plurality of sets may be combined from the differences in brightness, blinking speed, color, and size of the annotation image Q to differentiate the annotation images Q.

In the biological feature region arrangement developed image Eq, it is not necessary to display the plurality of annotation images Q in a superimposed manner. In the biological feature region arrangement developed image Eq, only the annotation image Q corresponding to the next feature region in the order may be displayed.

After step S20 ends, in step S21, observation display processing is executed with the next feature region in the order as a target. The processing of step S21 can be the processing of step S13 described above with the feature region in the next order as a target. As a result, the detail observation image D of the next feature region in the order is displayed on the display 22.

By visually recognizing the biological feature region arrangement developed image Eq and the detail observation image D displayed on the display 22, the user can observe the detail observation image D in detail while recognizing the position of the annotation image Q in the biological feature region arrangement developed image Eq.

In next step S22, the processing unit 30 determines the presence or absence of an inversion command (reverse command) of the focus order. When the inversion command is input through the user interface 24, 26 or the like, the processing proceeds to next step S23. When the inversion command is not input and it is determined that the inversion command is absent, the processing proceeds to step S17.

In step S23, processing of reversing the focus order is executed. For example, processing of reversing the order of the array defining the focus order is executed. The reversed focus order is stored as the focus order of subsequent processing. Thereafter, the processing proceeds to step S17. The determination of the presence or absence of the inversion command in step S22 may be made further upstream of the processing, for example, next to step S18, and if the inversion command is present, the processing in and after step S19 may be performed according to the instructed focus order.

By the processing of steps S19 to S21, the processing of altering the biological feature region arrangement developed image Eq displayed on the display 22 and changing the detail observation image D displayed on the display 22 is performed according to the focus order with the lapse of the preset time.

When the feature region is not designated and the automatic switching mode is not changed, the processing of steps S19 to S23 is repeated. Thereby, the biological feature regions can be observed in detail sequentially without omission and without duplication.

When it is determined in step S18 that the mode is not the automatic switching mode, the processing proceeds to step S24.

In step S24, the presence or absence of the next display command is determined. The next display command is a focus target switching command input through the user interface 24, 26. For example, the forward display command may be input by the rightward key or the downward key of the keyboard, and the backward display command may be input by the leftward key or the upward key. The processing of step S24 is repeated until it is determined that the next display command has been input regardless of the backward or forward display command, and when it is determined that the next display command has been input, the processing proceeds to next step S25.

In next step S25, the processing unit 30 determines whether the display command is a forward command. When it is determined that the display command is the forward command, the processing proceeds to step S26. In a case where the display command in the backward direction has been input, it is determined that the display command is not the forward command, and the processing proceeds to step S28.

In step S26, the processing unit 30 identifies, according to the focus order, the feature region that comes next in the order after the feature region being displayed, and alters the annotation image Q so that the next feature region in the order can be differentiated from other feature regions (See FIG. 16). The processing of step S26 can be similar to the processing of step S20 described above.

After step S26 ends, in step S27, observation display processing is executed with the next feature region in the order as a target. The processing of step S27 can be the processing similar to step S13 described above with the feature region in the next order as a target. As a result, the detail observation image D of the next feature region in the order is displayed on the display 22.

By visually recognizing the biological feature region arrangement developed image Eq and the detail observation image D displayed on the display 22, the user can observe the detail observation image D in detail while recognizing the position of the annotation image Q in the biological feature region arrangement developed image Eq.

In a case where it is determined in step S25 that the command is not the forward command, the processing proceeds to step S28.

In step S28, the processing unit 30 identifies the feature region that comes next in the reverse order after the feature region being displayed, that is, the previous feature region, according to the focus order. The annotation image Q is altered so that the previous feature region in the reverse order can be differentiated from other feature regions. The processing of step S28 can be similar to the processing of step S20 described above.

After step S28 ends, in step S29, observation display processing is executed with the previous feature region in the reverse order as a target. The processing of step S29 can be the processing similar to step S13 described above with the previous feature region in the reverse order as a target. As a result, the detail observation image D of the previous feature region in the reverse order is displayed on the display 22.

By visually recognizing the biological feature region arrangement developed image Eq and the detail observation image D displayed on the display 22, the user can observe the detail observation image D in detail while recognizing the position of the annotation image Q in the biological feature region arrangement developed image Eq.

If the feature region is not designated and the mode of not performing the automatic switching is not changed, the processing in and after step S24 is repeated, so that the annotation image Q alters and the detail observation image D alters according to the display command timing of the user. At this time, when the user gives a forward command, the biological feature regions are sequentially displayed without omission and without duplication according to the focus order.

At this time, in a case where the user wants to return and reconfirm the observed biological feature region, and the like, by the user giving a command in the reverse order direction, the display returns backward and the detail observation image D is displayed.

Note that, if the focus order data is defined such that the last biological feature region returns to the first biological feature region, the first biological feature region can be displayed according to the focus order after the last biological feature region is displayed.

In the above example, a case has been described where the processing of changing the biological feature region arrangement developed image and changing the detail observation image according to the focus order with the lapse of the preset time, and the processing performed in response to the focus target switching command input through the user interface 24, 26 are used together as different processing.

However, the processing performed with the lapse of the preset time and the processing performed in response to the focus target switching command may be combined. For example, on the premise of the processing of altering the biological feature region arrangement developed image and changing the detail observation image with the lapse of the preset time, when the focus target switching command is input even before the lapse of the time, the processing of altering the biological feature region arrangement developed image and changing the detail observation image in response to the command may be performed.

In addition, the processing of altering the biological feature region arrangement developed image and changing the detail observation image according to the focus order with the lapse of the preset time may be omitted, or the processing of altering the biological feature region arrangement developed image and changing the detail observation image according to the focus order in response to the focus target switching command may be omitted.

### <Effects and the like>

According to the image processing apparatus 20, the image processing method, and the program 34a configured as described above, it is indicated by the change in the annotation image Q that the biological feature regions are subjected to focusing in the focus order, and the detail observation image D corresponding to the focusing of the biological feature region is displayed on the display 22. Therefore, it is easy to sequentially recognize a plurality of biological feature regions by viewing the biological feature region arrangement developed image Eq. In addition, since the detail observation image D according to the focus order is displayed on the display 22, the focused biological feature region can be observed in detail. As compared with a case where the biological feature regions are individually designated, the biological feature regions can be easily recognized without omission and without duplication according to the focus order. Even in a case where a plurality of biological feature regions are respectively located adjacently, it is easy to recognize and observe the plurality of respective adjacent biological feature regions as separate biological feature regions.

In addition, the dentition developed image Ep is an image in which the entire region of the dentition constituent tissue E is viewed frontally, and the annotation image Q is displayed in the image. In this case, it is easy to recognize the position of the biological feature region in the entire region of the dentition constituent tissue E.

In addition, the biological feature region arrangement developed image Eq in which a plurality of annotation images Q are superimposed on the dentition developed image Ep is displayed on the display 22. Then, some annotation images targeted for focusing among the plurality of annotation images Q are displayed in a different manner from the rest of the annotation images Q. Therefore, the focused biological feature region can be observed in detail while easily recognizing the position of the focused biological feature region in the dentition developed image Ep.

At this time, if the annotation image Q targeted for focusing is displayed in a blinking manner, it is easy to recognize the focused biological feature region.

In addition, if the annotation image Q targeted for focusing is displayed at a different blinking speed, in a different color, or in a different size from other annotation images Q, the focused annotation image can be made noticeable.

If the focus order is determined on the basis of the plurality of biological feature region corresponding positions P2 in the dentition developed image Ep, the focus order can be determined on the basis of, for example, the width direction position or the up-down direction position of the biological feature region corresponding position. When the focus order is determined on the basis of positional regularity, it is easy to predict the focus order. Therefore, it is possible to sequentially observe the detail observation image D while predicting the focus order, and it is easy to perform the observation work.

When the biological feature region arrangement developed image Eq displayed on the display 22 is altered and the detail observation image D displayed on the display 22 is changed according to the focus order with the lapse of the preset time, the focus target can be automatically changed with the lapse of time.

Furthermore, it is convenient if the focus order can be changed reversely in response to the inversion command input through the user interface 24, 26. For example, it is possible to easily cope with a case where observation is performed too early or a case where it is desired to return backward for observation.

In addition, since the biological feature region arrangement developed image Eq displayed on the display 22 is altered and the detail observation image D displayed on the display 22 is changed according to the focus order in response to the focus target switching command, the focus target can be changed on the basis of the input to the user interface. Therefore, the detail observation image D can be changed according to the observation progress of the user. In addition, it is easy to differentiate even if there are adjacent biological feature regions.

In addition, if the detail observation image D includes three cross-sectional images Da, Db, Dc orthogonal to each other, it is easy to observe the biological feature region in detail.

In addition, the cross-sectional position of at least one of the three cross-sectional images Da, Db, Dc can be changed in response to the moving operation on the reference mark Li through the user interface 24, 26. Therefore, by changing the cross-sectional positions of the cross-sectional images Da, Db, Dc, it is easy to observe the biological feature region.

The position of the annotation image Q in the dentition developed image Ep does not change only by performing the moving operation on the reference mark Li, but the position of the annotation image Q in the dentition developed image Ep may be changed by receiving the change by performing the operation of changing the position of the annotation image Q after the moving operation.

The operation of changing the position of the annotation image Q may be performed by, for example, an operation on the detail observation image D. The operation may be performed by an operation using the reference mark Li. The change of the position of the annotation image Q may be referred to as annotation position change. The operation of the annotation position change may be referred to as annotation position change operation. The annotation position change operation is an operation of converting an operation on the X1Y1Z1 coordinates into a change in the annotation position on the RT coordinates or a change in the annotation position on the RST coordinates. Furthermore, the annotation position change operation is an operation for converting an operation on the captured image data three-dimensional image into a change in the annotation position on the dentition developed region coordinates.

Furthermore, the position of the annotation image Q in the dentition developed image Ep is changed in response to the moving operation on the reference mark Li through the user interface 24, 26. Therefore, it is easy to grasp at which position of the dentition developed image Ep the detail observation image after the moving operation is present.

In addition, since the detail observation image D includes the cross-sectional image Da in which the biological feature region is viewed frontally and the two cross-sectional images orthogonal to the cross-sectional image Da, it is easy to observe the biological feature region. In particular, by being based on the cross-sectional image Da in which the biological feature region is viewed frontally, the position and posture of the biological feature region with respect to the teeth can be easily grasped.

### <Modifications>

Various modifications will be described on the premise of the above preferred embodiment.

As in a first modification illustrated in FIG. 18, the processing unit 30 may determine the focus order on the basis of the importance levels of the biological feature regions.

For example, as described above, it is assumed that the biological feature region is the lesion region. The importance level of the lesion region may be determined on the basis of at least one of the degree of progression, the lesion site, and the risk and the certainty factor according to the disease name.

For example, the degree of progression is considered to have a positive correlation with the size of the detected biological feature region. Therefore, it is conceivable to make, using the size of the detected biological feature region as a parameter, the importance level higher as the biological feature region is larger.

For the lesion site, it is conceivable to increase the importance level as the lesion site is closer to the root of the tooth from the surface of the tooth. Therefore, based on the positional relationship between the detected biological feature region and the tooth or jawbone, the parameter can be used to identify the lesion site and determine the importance level.

Furthermore, regarding the disease name, for example, it is conceivable to increase the importance level in the case of cancer rather than dental caries and periodontal diseases. The estimation of the disease name of the biological feature region may be performed, for example, by applying a learned machine learning model. For example, as training data, a large number of pieces of data obtained by labeling the image data of the biological feature region with the disease name are prepared. A machine learning model learned to estimate a disease name of the biological feature region is prepared using the training data. By applying the learned machine learning model to the biological feature region, a disease name can be estimated.

In this case, since the focus order is determined according to the importance levels, the focus order may be randomly determined in the dentition developed image Ep as illustrated in FIG. 18. For example, when the feature regions are observed in the focus order in the dentition developed image Ep, the feature regions may be focused in the order from the bottom to the top or from the right to the left.

According to this modification, the focus order can be set according to the importance levels. Therefore, the detail observation images D can be observed in the order of conceivably higher importance levels.

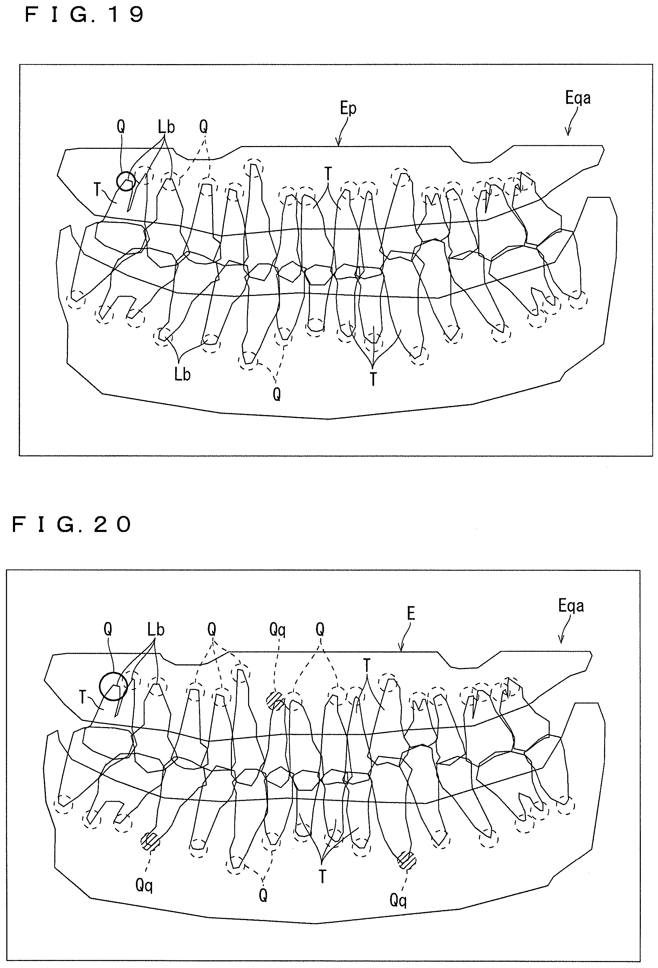

In the above preferred embodiment, an example in which the biological feature region is the lesion region L has been described. As in a biological feature region arrangement developed image Eqa of a second modification illustrated in FIG. 19, the biological feature region may be any region that is image-wise characteristic in the dentition constituent tissue E, and does not need to be the lesion region L. For example, the biological feature region may be a plurality of common biological feature regions having a common biological feature. Specifically, the biological feature region may be a root apex Lb of the tooth. In this case, in the dentition constituent tissue E, the annotation image Q is displayed at the root apex Lb of each of the plurality of teeth T. The focus order is determined for the plurality of root apexes Lb. The focus order is determined, for example, on the basis of the positions in the dentition constituent tissue E. For example, the focus order is determined such that the dentition above the lower dentition is ranked higher, and the right is ranked higher than the left.

As described in the above preferred embodiment, the annotation image Q alters according to the determined focus order so that the root apex Lb to be focused is identified. As a result, the user can sequentially observe the plurality of root apexes Lb in detail in the focus order while grasping respective positions.

According to the present modification, some of the plurality of annotation images Q are focused in the focus order in the plurality of common biological feature regions. Therefore, it is easy to perform observation in consideration of being a common biological feature.

Note that the common biological feature region may be a mandibular canal.

Note that, as in a third modification illustrated in FIG. 20, for the unique root apex Lb among the annotation images Q corresponding to the plurality of apexes Lb, an annotation image Qq different from other annotation images Q may be displayed regardless of whether it is focused or not. The unique root apex Lb is, for example, a portion where a lesion such as periodontal disease is detected. In this case, for example, the annotation image Qq and other annotation images Q may be indicated in different colors, in different shapes, at different blinking speeds, or in different sizes to each other.

For example, the annotation image Qq corresponding to the unique root apex Lb and the annotation images Q corresponding to other root apexes Lb may be displayed in a lighted manner in different colors to each other, and the focused root apex Lb may be displayed in a blinking manner. As a result, the plurality of root apexes Lb can be sequentially observed in detail while recognizing the unique root apex Lb.

Furthermore, the plurality of annotation images Q not targeted for focusing may be displayed in the same manner regardless of the presence or absence of uniqueness. Then, at the time of focusing, different display may be performed according to the presence or absence of uniqueness. For example, the annotation image Q that is not targeted for focusing is displayed in a blinking manner, and the focused annotation image Q is displayed in a blinking manner. At this time, the annotation image Q corresponding to the root apex Lb having no uniqueness may have the same color as that of the annotation image Q not targeted for focusing, and the annotation image Q corresponding to the root apex Lb having uniqueness may have the different color from that of the annotation image Q not targeted for focusing. As a result, the plurality of root apexes Lb can be sequentially observed in detail while recognizing the unique root apex Lb.

In the above preferred embodiment, an example has been described in which each position of the dentition constituent tissue E is identified on the basis of the three-dimensional volume data and developed in the dentition developed image Ep on the basis of the identified dentition constituent tissue E.

However, as a fourth modification, each position of the dentition constituent tissue E may not be individually identified on the basis of the three-dimensional volume data, and may be developed in the dentition developed image Ep on the basis of the region of the standard dentition constituent tissue E.

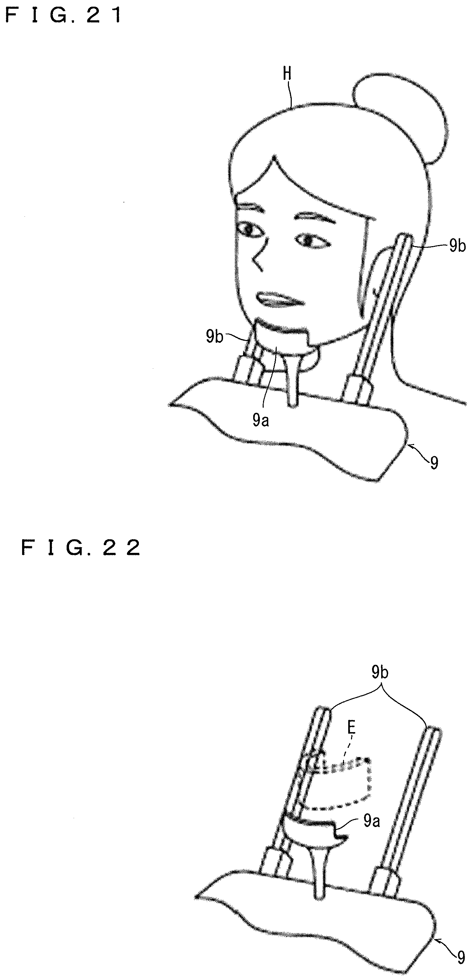

For example, as illustrated in FIG. 21, the head H is positioned at a fixed position by the head holder 9 when CT imaging is performed. As a result, as illustrated in FIG. 22, the dentition constituent tissue E is also positioned at a fixed position with respect to the head holder 9.

The positional relationship between the head holder 9 and the X-ray generator 11 and X-ray detector 12 can be a known positional relationship. In addition, the region in which the dentition constituent tissue E spreads in the head H may be considered to be constant to some extent. Therefore, in the three-dimensional volume data based on the CT imaging data, the region of the standard dentition constituent tissue E may be treated as the region of the actual dentition constituent tissue E.

In addition, on the premise of the standard dentition constituent tissue E, it is possible to determine in advance general-purpose conversion processing of developing the standard dentition constituent tissue E in the dentition developed image Ep. The region where the dentition constituent tissue E is assumed to be present in the three-dimensional volume data can be developed in the dentition developed image Ep by the general-purpose conversion processing. In addition, by performing general-purpose conversion processing on the detected biological feature region in the three-dimensional volume data, the corresponding position of the biological feature region in the dentition developed image Ep can be calculated.

In the above preferred embodiment, the case where the image processing apparatus 20 is used with the CT imaging apparatus 10 as a set has been described.

The image processing apparatus 20 may be configured as an apparatus separate from the CT imaging apparatus 10.

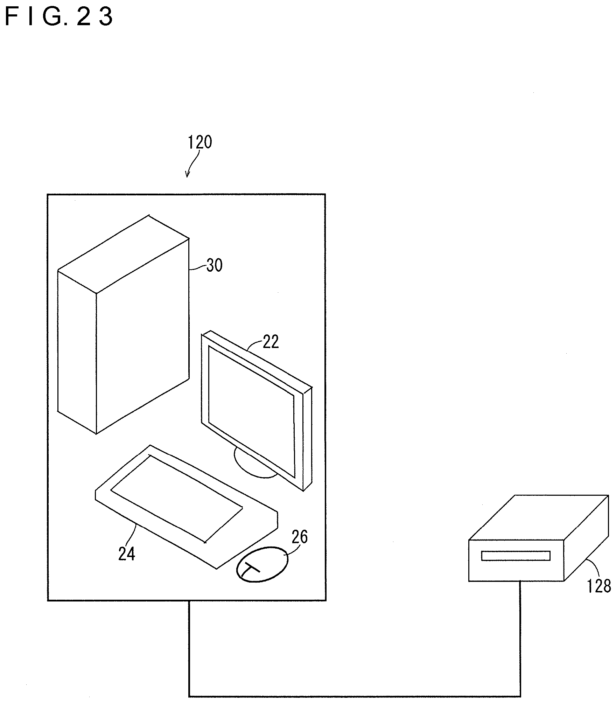

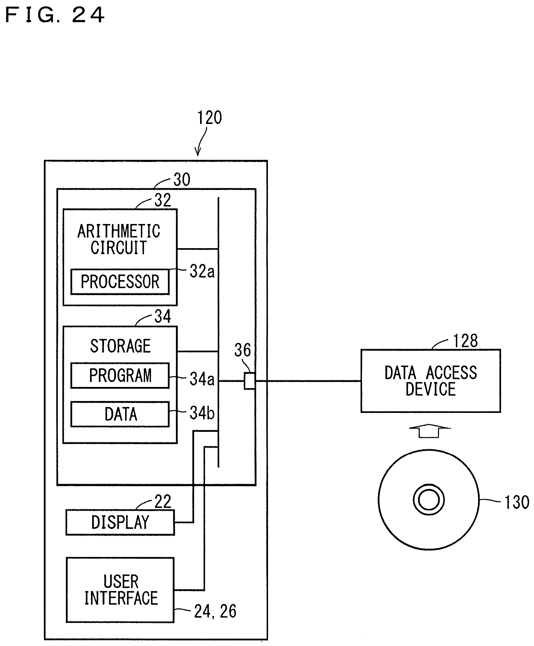

In this case, as in an image processing apparatus 120 illustrated in FIG. 23 and FIG. 24 according to the fifth modification, the image processing apparatus 120 may include a data access device 130 connected through the connection port 36. The data access device 130 is, for example, a device capable of reading data recorded on a recording medium 128. The recording medium 128 may be an optical recording medium such as an optical disk, or may be a flash memory such as a USB memory or an SD card. The data access device 130 may be a reading device for an optical disk, or a card reader.

Image data obtained by CT imaging is recorded in the recording medium 128. The image data may be an X-ray projection image data group, three-dimensional volume data, or a data group of a plurality of tomographic images.

The image processing apparatus 120 can execute processing similar to that of the above preferred embodiment by reading image data obtained by CT imaging via the data access device 130.

The image data obtained by the CT imaging is stored in the server apparatus, and the image processing apparatus 120 may access the server apparatus through the communication network to obtain the image data obtained by the CT imaging. In this case, the server apparatus may be located in a facility where the image processing apparatus 120 exists or may be located outside the facility. The server apparatus may be a cloud server. The image processing apparatus 120 can access the server apparatus through a dedicated line or a public line network to acquire image data obtained by CT imaging.

### {Other modifications}

In the above preferred embodiment and various modifications, other information may be displayed around the biological feature region arrangement developed image Eq or the detail observation image D. For example, a disease name may be described. For example, in a case where a plurality of lesion regions L are detected, the respective disease names of the plurality of lesion regions may be displayed. The disease names may be displayed together with the cross-sectional images.

Note that the configurations described in the above preferred embodiment and modifications can be appropriately combined as long as they do not contradict each other.

The present disclosure discloses the following aspects.

A first aspect is an image processing apparatus that processes image data obtained by computed tomography (CT) imaging of dentition constituent tissue to generate an image for diagnosis, the image processing apparatus including: a storage that stores the image data; a processor; and a display that displays an observation image on the basis of output of the processor, wherein the processor generates three-dimensional image data of the dentition constituent tissue on the basis of the image data, detects a plurality of biological feature regions in the dentition constituent tissue on the basis of the image data, identifies a plurality of three-dimensional positions at which the plurality of biological feature regions are respectively present in a coordinate system of the three-dimensional image data, generates a dentition developed image in which the dentition constituent tissue is developed on the basis of the three-dimensional image data, identifies a plurality of biological feature region corresponding positions respectively corresponding to the plurality of three-dimensional positions in the dentition developed image, displays, on the display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions, generates detail observation images at the plurality of three-dimensional positions, respectively, on the basis of the three-dimensional image data, determines a focus order of the plurality of biological feature regions according to a predetermined rule, and when displaying the biological feature region arrangement developed image, indicates that the biological feature regions are subjected to focusing in the focus order by a change in the annotation images and displays, on the display, the detail observation images corresponding to focusing of the biological feature regions.

According to this image processing apparatus, it is indicated by the change in the annotation images that the biological feature regions are subjected to focusing in the focus order, and the detail observation images corresponding to the focusing of the biological feature regions are displayed on the display. Therefore, it is easy to sequentially recognize the plurality of biological feature regions by viewing the biological feature region arrangement developed image. In addition, since the detail observation images according to the focused image are displayed on the display, the focused biological feature region can be observed in detail. Therefore, the plurality of biological feature regions are easily recognized without omission.

A second aspect is the image processing apparatus according to the first aspect, wherein the processor displays, on the display, the biological feature region arrangement developed image in which the plurality of annotation images are superimposed on the dentition developed image, and displays some annotation images targeted for the focusing among the plurality of annotation images in a manner different from the rest of the annotation images.

In this case, since the annotation image targeted for focusing is displayed in a different manner from other annotation images, it is possible to observe the focused biological feature region in detail while easily recognizing the position of the focused biological feature region.

A third aspect is the image processing apparatus according to the first or second aspect, wherein the processor displays some annotation images targeted for the focusing among the plurality of annotation images in a blinking manner.

As a result, the annotation image targeted for focusing is displayed in a blinking manner, so that it is easy to recognize the focused biological feature region.

A fourth aspect is the image processing apparatus according to the first or second aspect, wherein the processor displays some annotation images targeted for the focusing among the plurality of annotation images at a different blinking speed, in a different color, or in a different size from the rest of annotation images.

As a result, by displaying the focused annotation image at a different blinking speed, in a different color, or in a different size from other annotation images, the focused annotation image can be made noticeable.

A fifth aspect is the image processing apparatus according to any one of the first to fourth aspects, further including a user interface, wherein the processor alters the biological feature region arrangement developed image displayed on the display according to the focus order in response to a focus target switching command input through the user interface, and changes the detail observation images displayed on the display.

As a result, the focus target can be changed on the basis of the input to the user interface.

A sixth aspect is the image processing apparatus according to any one of the first to fifth aspects, wherein the processor alters the biological feature region arrangement developed image displayed on the display according to the focus order with a lapse of a preset time, and changes the detail observation images displayed on the display.

As a result, the focus target can be changed with the lapse of time.

A seventh aspect is the image processing apparatus according to any one of the first to sixth aspects, further including a user interface, wherein the processor changes the focus order reversely in response to a reverse command input through the user interface.

This is convenient because the focus order can be changed reversely.

An eighth aspect is the image processing apparatus according to any one of the first to seventh aspects, wherein the processor determines the focus order on the basis of the plurality of biological feature region corresponding positions in the dentition developed image.

As a result, the focus order can be determined on the basis of the plurality of biological feature region corresponding positions in the dentition developed image. For example, the focus order can be determined on the basis of the width direction position or the up-down direction position of the biological feature region corresponding positions.

A ninth aspect is the image processing apparatus according to any one of the first to eighth aspects, wherein the processor detects, as the plurality of biological feature regions, a plurality of common biological feature regions having a common biological feature.

As a result, some of the plurality of annotation images are focused in the focus order in the plurality of common biological feature regions. Therefore, it is easy to perform observation in consideration of being a common biological feature.

A tenth aspect is the image processing apparatus according to any one of the first to ninth aspects, wherein the processor sets importance levels for the plurality of biological feature regions, respectively, on the basis of respective features of the biological feature regions, and determines the focus order on the basis of the importance levels of the plurality of biological feature regions.

As a result, the focus order can be set according to the importance levels.

An eleventh aspect is the image processing apparatus according to any one of the first to tenth aspects, wherein the processor generates, as the dentition developed image, an image in which an entire region of the dentition constituent tissue is viewed frontally.

As a result, the annotation image indicating the biological feature region is displayed in the image in which the entire region of the dentition constituent tissue is viewed frontally, so that it is easy to recognize the position of the biological feature region in the entire region of the dentition constituent tissue.

The processor may set dentition constituent tissue region that is a region in which the dentition constituent tissue spreads and a dentition developed region in which the dentition constituent tissue region is developed, and the development of the dentition constituent tissue may be performed by development processing of developing image data of the dentition constituent tissue in the dentition constituent tissue region so as to conform to a shape of the dentition developed region. As a result, appropriate image data for visual recognition of the biological feature region can be processed.

The development processing may be performed such that the buccolingual direction in the pre-development curved state, which is the state of the dentition constituent tissue region before development, corresponds to the frontally-viewing direction in the post-development at-a-glance state, which is the state of the dentition developed region after development. This facilitates positional understanding of the biological feature region.

Setting may be made such that the dentition constituent tissue region has a thickness in the buccolingual direction and the dentition developed region has a thickness in the frontally-viewing direction corresponding to the buccolingual direction. As a result, it is possible to recognize a biological feature region within an appropriate range.

In the development described above, an original coordinates arithmetic operation for associating the biological feature region with the three-dimensional position of the biological feature region corresponding position may be performed. In the original coordinates arithmetic operation, arithmetic operation of identifying which position in the buccolingual direction the three-dimensional position of the biological feature region is located and identifying which position in the frontally-viewing direction corresponding to the buccolingual direction the biological feature region corresponding position is located may be performed. As a result, it is possible to calculate with accuracy the three-dimensional position of the biological feature region and the biological feature region corresponding position.

A twelfth aspect is the image processing apparatus according to any one of the first to eleventh aspects, wherein the detail observation image includes three cross-sectional images orthogonal to each other.

This makes it easy to observe the biological feature region in detail.

A thirteenth aspect is the image processing apparatus according to the twelfth aspects, further including a user interface, wherein each of the three cross-sectional images includes a reference mark indicating positions of the other cross sections, and the processor changes a cross-sectional position of at least one of the three cross-sectional images in response to a moving operation on the reference mark through the user interface.

As a result, by changing the cross-sectional positions of the detail observation image, it is easy to observe the biological feature region.

The processor may perform processing of differentiating a dentition region and an alveolar bone region on the basis of the three-dimensional image data, and perform image processing in which the dentition region and the alveolar bone region are displayed in different manners as the dentition developed image. As a result, the dentition region and the alveolar bone region are displayed in a differentiated manner in the dentition developed image, so that it is easy to grasp the position of the biological feature region.

The processor may cause the dentition region and the alveolar bone region to have different colors in the dentition developed image. As a result, it is easy to differentiate and recognize the dentition region and the alveolar bone region.

The processor may set transparency to the alveolar bone region to such an extent that the dentition region can be seen through. As a result, also for the portion of the dentition present inside the alveolar bone, it is possible to observe the biological feature region while grasping the positional relationship between the alveolar bone and the dentition.

The processor may indicate functional dentition constituent tissue, which is function-specific tissue constituting dentition constituent tissue of a living body, by a boundary surface between presence and absence, and perform line-of-sight direction-corresponding transparency image processing, which is image processing in which transparency changes depending on the line-of-sight direction, on the boundary surface. As a result, it is easy to visually recognize the shape of the functional dentition constituent tissue.

When the element increasing the transparency is the pro-transparency element, increasing the transparency is the pro-transparency, the element for decreasing the transparency is the anti-transparency element, and decreasing the transparency is the anti-transparency, the line-of-sight direction-corresponding transparency image processing may be a combination of at least any one of the following group A with at least any one of the following group B.
Group A:
   - Processing of enlarging or increasing the pro-transparency element of the frontally-viewed boundary surface described above
   - Processing of promoting pro-transparency of the frontally-viewed boundary surface described above
   - Processing of downsizing, or reducing or eliminating the anti-transparency element of the frontally-viewed boundary surface described above
   - Processing of suppressing anti-transparency of the frontally-viewed boundary surface described above
Group B:
   - Processing of downsizing, or reducing or eliminating the pro-transparency element of the obliquely-viewed or laterally-viewed boundary surface described above
   - Processing of suppressing pro-transparency of the obliquely-viewed or laterally-viewed boundary surface described above

   - Processing of enlarging or increasing the anti-transparency element of the obliquely-viewed or laterally-viewed boundary surface described above
   - Processing of promoting anti-transparency of the obliquely-viewed or laterally-viewed boundary surface described above
   As a result, the three-dimensional shape of the functional dentition constituent tissue is easily visually recognized.

A fourteenth aspect is an image processing method of processing image data obtained by computed tomography (CT) imaging of dentition constituent tissue to generate an image for diagnosis, the method including: generating three-dimensional image data of the dentition constituent tissue on the basis of the image data; detecting a plurality of biological feature regions in the dentition constituent tissue on the basis of the image data; identifying a plurality of three-dimensional positions at which the plurality of biological feature regions are respectively present in a coordinate system of the three-dimensional image data; generating a dentition developed image in which the dentition constituent tissue is developed on the basis of the three-dimensional image data; identifying a plurality of biological feature region corresponding positions respectively corresponding to the plurality of three-dimensional positions in the dentition developed image; displaying, on a display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions; generating detail observation images at the plurality of three-dimensional positions, respectively, on the basis of the three-dimensional image data; determining a focus order of the plurality of biological feature regions according to a predetermined rule; and when displaying the biological feature region arrangement developed image, indicating that the biological feature regions are subjected to focusing in the focus order by a change in the annotation images and displaying, on the display, the detail observation images corresponding to focusing of the biological feature regions.

According to this image processing method, it is indicated by the change in the annotation image that the biological feature regions are subjected to focusing in the focus order, and the detail observation images corresponding to the focusing of the biological feature regions are displayed on the display. By viewing the biological feature region arrangement developed image, it is easy to sequentially recognize a plurality of biological feature regions. In addition, since the detail observation images according to the focused image are displayed on the display, the focused biological feature region can be observed in detail. Therefore, it is easy to recognize the plurality of biological feature regions without omission.

A fifteenth aspect is a program for processing image data obtained by computed tomography (CT) imaging of dentition constituent tissue to generate an image for diagnosis, the program being configured to cause a computer to execute processing of: generating three-dimensional image data of the dentition constituent tissue on the basis of the image data; detecting a plurality of biological feature regions in the dentition constituent tissue on the basis of the image data; identifying a plurality of three-dimensional positions at which the plurality of biological feature regions are respectively present in a coordinate system of the three-dimensional image data; generating a dentition developed image in which the dentition constituent tissue is developed on the basis of the three-dimensional image data; identifying a plurality of biological feature region corresponding positions respectively corresponding to the plurality of three-dimensional positions in the dentition developed image; displaying, on a display, a biological feature region arrangement developed image in which at least one of a plurality of annotation images respectively indicating the plurality of biological feature regions is superimposed on the dentition developed image such that the annotation images are located at corresponding positions of the plurality of biological feature region corresponding positions; generating detail observation images at the plurality of three-dimensional positions, respectively, on the basis of the three-dimensional image data; determining a focus order of the plurality of biological feature regions according to a predetermined rule; and when displaying the biological feature region arrangement developed image, indicating that the biological feature regions are subjected to focusing in the focus order by a change in the annotation images and displaying, on the display, the detail observation images corresponding to focusing of the biological feature regions.

According to this program, it is indicated by the change in the annotation image that the biological feature regions are subjected to focusing in the focus order, and the detail observation image corresponding to the focusing of the biological feature region is displayed on the display. Therefore, it is easy to sequentially recognize the plurality of biological feature regions by viewing the biological feature region arrangement developed image. In addition, since the detail observation images according to the focused image are displayed on the display, the focused biological feature region can be observed in detail. Therefore, it is easy to recognize the plurality of biological feature regions without omission.

The above description is illustrative in all phases, and the present invention is not limited thereto. It is understood that numerous modifications not illustrated can be assumed without departing from the scope of the present invention.

While the disclosure has been shown and described in detail, the foregoing description is in all aspects illustrative and not restrictive. It is therefore understood that numerous modifications and variations can be devised.

## Claims

1. An image processing apparatus that processes image data obtained by computed tomography imaging of dentition constituent tissue to generate an image for diagnosis, the image processing apparatus comprising:
a storage that stores said image data;
a processor; and
a display that displays an observation image on the basis of output of said processor,
wherein said processor
generates three-dimensional image data of said dentition constituent tissue on the basis of said image data,
detects a plurality of biological feature regions in said dentition constituent tissue on the basis of said image data,
identifies a plurality of three-dimensional positions at which said plurality of biological feature regions are respectively present in a coordinate system of said three-dimensional image data,
generates a dentition developed image in which said dentition constituent tissue is developed on the basis of said three-dimensional image data,
identifies a plurality of biological feature region corresponding positions respectively corresponding to said plurality of three-dimensional positions in said dentition developed image,
displays, on said display, a biological feature region arrangement developed image in which at least one of a plurality of annotation image respectively indicating said plurality of biological feature regions is superimposed on said dentition developed image such that said annotation images are located at corresponding positions of said plurality of biological feature region corresponding positions,
generates detail observation images at said plurality of three-dimensional positions, respectively, on the basis of said three-dimensional image data,
determines a focus order of said plurality of biological feature regions according to a predetermined rule, and
when displaying said biological feature region arrangement developed image, indicates that said biological feature regions are subjected to focusing in said focus order by a change in said annotation images and displays, on said display, said detail observation images corresponding to focusing of said biological feature regions.

2. The image processing apparatus according to claim 1, wherein
said processor
displays, on said display, said biological feature region arrangement developed image in which said plurality of annotation images are superimposed on said dentition developed image, and
displays some annotation images targeted for focusing among said plurality of annotation images in a manner different from the rest of said annotation images.

3. The image processing apparatus according to claim 1 or claim 2, wherein
said processor displays some annotation images targeted for focusing among said plurality of annotation images in a blinking manner.

4. The image processing apparatus according to claim 1 or claim 2, wherein
said processor displays some annotation images targeted for focusing among said plurality of annotation images at a different blinking speed, in a different color, or in a different size from said rest of annotation images.

5. The image processing apparatus according to claim 1 or claim 2, further comprising a user interface,
wherein said processor alters the biological feature region arrangement developed image displayed on said display according to said focus order in response to a focus target switching command input through said user interface, and changes said detail observation images displayed on said display.

6. The image processing apparatus according to claim 1 or claim 2, wherein
said processor alters said biological feature region arrangement developed image displayed on said display according to said focus order with a lapse of a preset time, and changes said detail observation images displayed on said display.

7. The image processing apparatus according to claim 1 or claim 2, further comprising a user interface,
wherein said processor changes said focus order reversely in response to a reverse command input through said user interface.

8. The image processing apparatus according to claim 1 or claim 2, wherein
said processor determines said focus order on the basis of said plurality of biological feature region corresponding positions in said dentition developed image.

9. The image processing apparatus according to claim 1 or claim 2, wherein
said processor detects, as said plurality of biological feature regions, a plurality of common biological feature regions having a common biological feature.

10. The image processing apparatus according to claim 1 or claim 2, wherein
said processor
sets importance levels for said plurality of biological feature regions, respectively, on the basis of respective features of said biological feature regions, and
determines said focus order on the basis of the importance levels of said plurality of biological feature regions.

11. The image processing apparatus according to claim 1 or claim 2, wherein
said processor generates, as said dentition developed image, an image in which an entire region of said dentition constituent tissue is viewed frontally.

12. The image processing apparatus according to claim 1 or claim 2, wherein
said detail observation image includes three cross-sectional images orthogonal to each other.

13. The image processing apparatus according to claim 12, further comprising a user interface,
wherein each of said three cross-sectional images includes a reference mark indicating positions of the other cross sections, and
said processor changes a cross-sectional position of at least one of said three cross-sectional images in response to a moving operation on said reference mark through the user interface.

14. An image processing method of processing image data obtained by computed tomography imaging of dentition constituent tissue to generate an image for diagnosis, the method comprising:
generating three-dimensional image data of said dentition constituent tissue on the basis of said image data;
detecting a plurality of biological feature regions in said dentition constituent tissue on the basis of said image data;
identifying a plurality of three-dimensional positions at which said plurality of biological feature regions are respectively present in a coordinate system of said three-dimensional image data;
generating a dentition developed image in which said dentition constituent tissue is developed on the basis of said three-dimensional image data;
identifying a plurality of biological feature region corresponding positions respectively corresponding to said plurality of three-dimensional positions in said dentition developed image;
displaying, on a display, a biological feature region arrangement developed image in which at least one of a plurality of annotation images respectively indicating said plurality of biological feature regions is superimposed on said dentition developed image such that said annotation images are located at corresponding positions of said plurality of biological feature region corresponding positions;
generating detail observation images at said plurality of three-dimensional positions, respectively, on the basis of said three-dimensional image data;
determining a focus order of said plurality of biological feature regions according to a predetermined rule; and
when displaying said biological feature region arrangement developed image, indicating that said biological feature regions are subjected to focusing in said focus order by a change in said annotation images and displaying, on said display, said detail observation images corresponding to focusing of said biological feature regions.

15. A program for processing image data obtained by computed tomography imaging of dentition constituent tissue to generate an image for diagnosis, the program being configured to cause a computer to execute processing of:
generating three-dimensional image data of said dentition constituent tissue on the basis of said image data;
detecting a plurality of biological feature regions in said dentition constituent tissue on the basis of said image data;
identifying a plurality of three-dimensional positions at which said plurality of biological feature regions are respectively present in a coordinate system of said three-dimensional image data;
generating a dentition developed image in which said dentition constituent tissue is developed on the basis of said three-dimensional image data;
identifying a plurality of biological feature region corresponding positions respectively corresponding to said plurality of three-dimensional positions in said dentition developed image;
displaying, on a display, a biological feature region arrangement developed image in which at least one of a plurality of annotation images respectively indicating said plurality of biological feature regions is superimposed on said dentition developed image such that said annotation images are located at corresponding positions of said plurality of biological feature region corresponding positions;
generating detail observation images at said plurality of three-dimensional positions, respectively, on the basis of said three-dimensional image data;
determining a focus order of said plurality of biological feature regions according to a predetermined rule; and
when displaying said biological feature region arrangement developed image, indicating that said biological feature regions are subjected to focusing in said focus order by a change in said annotation images and displaying, on said display, said detail observation images corresponding to focusing of said biological feature regions.
